# EUROPEAN PATENT APPLICATION

(11) **EP 4 702 951 A1**
(43) Date of publication of application: **04.03.2026**
(21) Application number: 24796155.0
(22) Date of filing: 25.04.2024
(51) Int. Cl.: A61F 2/24, A61F 2/95

(54) **CONTROL MECHANISM, LOCKING MECHANISM, LOADING METHOD, AND PRE-LOADING METHOD OF ARTIFICIAL IMPLANT**

(30) Priority: 26.04.2023 CN 202310466541; 26.04.2023 CN 202310470457; 17.08.2023 CN 202311041416; 17.08.2023 CN 202311047853; 11.04.2024 CN 202410435651
(71) Applicant: Venus MedTech (HangZhou) Inc., Binjiang District Hangzhou Zhejiang 310052 (CN); Hangzhou Qianjiang Medical and Industrial Cross Innovation Technology Research Institute, Hangzhou, Zhejiang 310052 (CN)
(72) Inventor: LEI, Rongjun, Hangzhou, Zhejiang 310052 (CN); CHEN, Wei, Hangzhou, Zhejiang 310052 (CN); YANG, Lingfeng, Hangzhou, Zhejiang 310052 (CN); WANG, Xiang, Hangzhou, Zhejiang 310052 (CN)
(74) Representative: Metida
(86) International application number: PCT/CN2024/089744
(87) International publication number: WO 2024/222779

(57) **Abstract**

Disclosed in the present application are a control mechanism, a locking mechanism, a loading method, and a pre-loading method for an artificial implant. The control mechanism for the artificial implant includes a base, a pulling wire, and a locking member. The base is configured with a locking hole. The pulling wire has a free end that can be passed through, wound around, or detached from the artificial implant. The locking member and the base are rotatably engaged to lock the free end of the pulling wire. After the control mechanism is optimized, it can cooperate to realize the control of the expansion, release, or withdrawal process of the artificial implant in the body, which is more suitable for internal work than existing structures.

## Description

### TECHNICAL FIELD

This application relates to the technical field of medical devices, and in particular to a control mechanism, locking mechanism, loading method, and pre-loading method for an artificial implant.

### BACKGROUND

With the development of medical conditions, artificial implants have been used to treat heart valve disorders. The treatment methods generally include repairing or replacing the valve through surgery, or using a flexible catheter to intervene and implant an artificial implant into the human body.

In interventional technology, a compressed and loaded artificial implant is installed at the distal end of a catheter assembly and delivered to a predetermined site. After the artificial implant is expanded radially and released, the catheter assembly is withdrawn from the human body.

### SUMMARY

### TECHNICAL PROBLEM

For the connection between the artificial implant and catheter assembly, the existing methods that can be used are direct clamping or wire control. The wire control method uses a flexible component to bind the artificial implant to the distal end of the catheter assembly and locks the flexible component to maintain the compressed state of the artificial implant. When it is necessary to release the artificial implant, the flexible component is unlocked and then released until it separates from the artificial implant to complete the release of the artificial implant. However, the existing control methods and structures for the flexible component still need to be improved.

### TECHNICAL SOLUTION

This application provides a control mechanism for an artificial implant, which enables more reasonable control (including locking and unlocking) of flexible component. The present application provides a control mechanism for an artificial implant, including:
a base configured with a locking area;
a pulling wire having a free end being capable of passing through or of being detached therefrom; and
a locking member being rotatably engaged with the base, wherein the locking member includes a positioning portion that engages into or disengages from the locking area during its rotation, and the positioning portion cooperates with the free end of the pulling wire to restrict the artificial implant.

Optionally, the base has a distal end and a proximal end opposite to each other, and an axial direction extending between the distal end and proximal end, an interior of the base defines a first cavity, at least a part of the locking member is configured as a connecting portion located in the first cavity, and the positioning portion further extends from the connecting portion to the distal end.

Optionally, the base has a distal end and a proximal end opposite to each other, and an axial direction extending between the distal end and proximal end, at least a part of the locking member is configured as a connecting portion located at a distal end side of the base, and the positioning portion further extends from the connecting portion to the proximal end.

The present application further provides a control mechanism for an artificial implant, including:
a base having a locking hole;
a pulling wire having a free end being capable of passing through and winding around the artificial implant or of being detached therefrom; and
a locking member being rotatably engaged with the base, wherein, during rotation, the locking member engages or disengages the locking hole, and the locking member cooperates with the base to lock or unlock the free end of the pulling wire.

The present application further provides a control mechanism for an artificial implant, including:
a base having a locking hole;
a pulling wire having a free end being capable of passing through and winding around the artificial implant or of being detached therefrom; and
a locking member in whole being located at a distal end of the base, and the locking member being rotatably engaged with the base to lock or unlock the free end of the pulling wire.

Optionally, the locking member and the base have a locking state in which they cooperate with each other and an unlocking state in which the cooperation is released, and in the unlocking state, the locking member is capable of further moving towards the distal end of the base relative to the locking state.

Optionally, the base is configured with a plurality of locking holes which are arranged offset from each other in an axial direction.

Optionally, the plurality of locking holes are arranged in sequence along a circumferential direction.

Optionally, the plurality of locking holes are located at different positions in an axial direction.

Optionally, the plurality of locking holes are arranged along a helical path.

Optionally, the locking member generates an axial displacement during its rotation and enters each locking hole sequentially.

Optionally, the locking member and the base have a locking state in which they cooperate with each other and an unlocking state in which the cooperation is released, and compared to the locking state, the locking member in the unlocking state further moves towards the distal end during its rotation.

The present application further provides a control mechanism for an artificial implant, including:
a base, including a plurality of components fixed to each other, wherein two of the plurality of components are enclosed at their joint to form the locking hole;
a pulling wire having a free end being capable of passing through and winding around the artificial implant or of being detached therefrom; and
a locking member being rotatably engaged with the base, wherein, during rotation, the locking member engages or disengages the locking hole to lock or unlock the free end of the pulling wire.

Optionally, the base has a radial direction, at least parts of the two of the plurality of components are in contact with each other in the radial direction, and the locking hole is located at a position where the two of the plurality of components are in contact in the radial direction.

Optionally, the base has an axial direction, at least parts of the two of the plurality of components are in contact with each other in the axial direction, and the locking hole is located at a position where the two of the plurality of components are in contact in the axial direction.

The present application further provides a control mechanism for an artificial implant, including:
a base including an inner cylinder and an outer cylinder mounted around and fixed to the inner cylinder, and a locking hole being provided radially between the outer cylinder and the inner cylinder;
a pulling wire having a free end being capable of passing through and winding around the artificial implant or of being detached therefrom; and
a locking member being rotatably engaged with the base, wherein, during rotation, the locking member engages or disengages the locking hole to lock or unlock the free end of the pulling wire.

Optionally, the base has a distal end and a proximal end opposite to each other, and an axial direction extending between the distal end and proximal end, an interior of the base defines a first cavity, a proximal end side of the base defines a first opening communicating with the first cavity, and an outer circumferential surface of the base defines a second opening communicating with the first cavity.

Optionally, the locking hole intersects with the second opening, and the locking member enters or exits the locking hole at an intersection of the locking hole and the second opening.

Optionally, the locking hole intersects with the second opening, and an intersection of the locking hole and the second opening serves as an open side of the locking hole for the locking member to engage into or disengage from the locking hole. Optionally, the inner cylinder is an axial through structure to form the first cavity.

Optionally, the plurality of locking holes are arranged along a helical path which intersects with the second opening, and all of the locking holes are communicated with each other through the second opening directly or indirectly.

Optionally, an inner wall of the outer cylinder and/or an outer wall of the inner cylinder is/are configured with a helical groove for providing the locking holes, and the helical groove intersects with the second opening.

Optionally, the outer cylinder and the inner cylinder each are made of metal or plastic.

Optionally, a proximal end side of the outer cylinder is configured as an extension section extending beyond the inner cylinder, and the extension section is configured to connect a third shaft which extends towards a proximal end.

Optionally, a side wall of the extension section is configured with a connecting hole for connecting the third shaft.

Optionally, positioning structures that cooperates with each other are provided between the outer cylinder and the inner cylinder.

Optionally, an outer wall of the inner cylinder is configured with a positioning groove, and an inner wall of the outer cylinder is configured with a positioning block which is engaged into the positioning groove. Optionally, a part of a side wall of the outer cylinder deforms to form the positioning block.

Optionally, the helical groove extends to a distal end surface of the inner cylinder.

Optionally, a portion of the helical groove at the distal end surface of the inner cylinder is configured with a guiding slope to guide the locking member to be assembled into the helical groove.

Optionally, there is plurality of second openings arranged at intervals along a circumferential direction of the base.

Optionally, an orientation of an open direction of the locking hole is the circumferential direction of the base.

Optionally, there are plurality of locking holes, and the locking member engages or disengages the locking holes in sequence.

Optionally, the control mechanism further includes a first shaft configured for driving the locking member to rotate relative to the base.

Optionally, a distal end of the first shaft movably penetrates the base, and the locking member selectively linked with the first shaft. Optionally, the locking member is movably mounted around the first shaft.

Optionally, the pulling wire further includes a control end opposite to the free end, the control end is movable relative to the base, and the control mechanism further includes a second shaft connected to the control end.

Optionally, the second shaft is tubular-shaped and movably mounted around the first shaft.

Optionally, the control mechanism further includes a third shaft, the base is connected to a distal end of the third shaft.

Optionally, the third shaft is movably mounted around the first shaft.

Optionally, the second shaft is slidably mounted around the first shaft, and the third shaft is slidably mounted around the second shaft.

Optionally, the control mechanism further includes three shafts, a second shaft is slidably mounted around a first shaft, and a third shaft is slidably mounted around the second shaft, the base is connected to a distal end of the third shaft, the pulling wire is connected to a distal end of the second shaft, and the locking member is connected to a distal end of the first shaft in a selectively linked manner.

Optionally, a loading section is fixed to a distal end of the first shaft and opens towards a proximal end, configured for accommodating a distal portion of the artificial implant; the loading section is configured as a radially deformable structure to adapt to being wrapped by the artificial implant when the artificial implant is withdrawn.

Optionally, the base is configured with a locking area, and the locking member includes a positioning portion that enters or exits the locking area during its rotation, and the positioning portion cooperates with the free end of the pulling wire to restrict the artificial implant.

Optionally, the pulling wire extends through the first cavity, one end of the pulling wire extends out of the base through the first opening towards the proximal end, and another end of the pulling wire (the free end) extends out of the base through the second opening for connecting the artificial implant.

Optionally, the locking area is located in the second opening. For example, there is a plurality of second openings arranged at intervals along a circumferential direction of the base, and ribs are provided between adjacent second openings
Optionally, all of the ribs are converged and fixed to each other at a distal end of the base. For example, all of the ribs are converged to form an annular structure.

Optionally, at least one of the ribs is provided with the locking hole. Optionally, an orientation of an open direction of the locking hole is the circumferential direction of the base. Optionally, all of the ribs are configured with the locking hole.

Optionally, there is a plurality of ribs configured with the locking hole, and the positioning portion as a whole has a tail end and a head end that are opposite to each other, and the head end is engaged into or disengaged from each locking hole sequentially along with the rotation of the locking member. For example, there is/are one locking hole or a plurality of locking holes on the same rib.

Optionally, the locking holes are arranged in sequence along an extension direction of the rib where they are located.

Optionally, the free end of the pulling wire has a first state being restricted to the base and a second state being released from the base, and when the cooperation between the locking member and the free end of the pulling member is released, a restriction on the artificial implant is released;
in the first state, the free end extends out of the base through a corresponding second opening, winds around the artificial implant, and returns to the locking area corresponding to the same second opening or returns to the locking area corresponding to an adjacent second opening.

Optionally, the free end of the pulling wire in the first state is located in a current locking area, and two ends of the positioning portion located in the current locking area are inserted into the locking holes at corresponding sides, respectively.

Optionally, along the circumferential direction of the base, there is a plurality of locking areas arranged at intervals, and the positioning portion enters or exits the locking areas in sequence along with the movement of the locking member.

Optionally, the locking member includes a positioning portion that enters or exits the locking area during its rotation, and the positioning portion cooperates with the free end of the pulling wire to restrict the artificial implant; the positioning portion has a curved shape and extends circumferentially around the base, and the positioning portion is engaged into or disengaged from the locking hole along with the rotation of the locking member.

Optionally, the positioning portion includes at least one arc-shaped structure that matches with the base.

Optionally, the positioning portion is configured as a helical structure. Optionally, the helical structure is wound at least one turn.

Optionally, the helical structure is configured with a plurality of turns which are arranged along an axial direction.

Optionally, the helical structure is a cylindrical helical or at least partially a conical helical.

Optionally, the positioning portion as a whole has a tail end and a head end being opposite to each other along a winding direction of the helical structure, and a connecting portion is fixed to one of the tail end and head end of the positioning portion.

Optionally, the connecting portion is tubular-shaped, and a distal end of the positioning portion is mounted around an outer circumference of the connecting portion or abuts against an end portion of the connecting portion.

Optionally, the connecting portion 135 is movably mounted around the distal end of the first shaft 21, and the connecting portion 135 can be axially separated from the distal end of the first shaft 21. Optionally, the free end of the pulling wire has a first state being restricted to the base and a second state being released from the base, when the cooperation between the locking member and the free end of the pulling member is released, a restriction on the artificial implant is released.

Optionally, in the first state, a limit mechanism is provided between the locking member and the base to prevent the locking member from rotating in an insertion direction. Optionally, the limit mechanism includes a first end surface provided at a proximal end of the connecting portion, and a second end surface provided at a distal end of the base, and the second end surface abuts the first end surface. Abutment between the first end surface and the second end surface limit the rotation and axial movement of the locking member. Optionally, the connecting portion is tubular-shaped with constant diameter or at least expands radially and outwardly at its proximal end, the proximal end of the connecting portion is configured as a connecting section, a distal end of the base includes an extending section, and the connecting section and the extending section are nested with each other.

Optionally, the free end of the pulling wire has a first state being restricted to the base and a second state being released from the base; the pulling wire further includes a control end opposite to the free end, the control end is movable relative to the base; when the free end is in the first state, a length of the pulling wire exposed out of the base is adjusted by operating the control end; and when the free end is in the second state, the pulling wire is driven to detach from the artificial implant by operating the control end.

Optionally, the pulling wire further includes a control end opposite to the free end, and multiple pulling wires are configured with the free ends move independently of each other, while the control ends move synchronously.

Optionally, the control ends of the pulling wires are connected to a second shaft.

Optionally, the free end has a ring, when the free end is in the first state, the positioning portion extends into the ring, and when the free end is in the second state, the positioning portion exits the ring.

Optionally, the ring is independently configured or wound by the pulling wire.

Optionally, the positioning portion is a helical structure with a plurality of turns, and there is an axial gap between adjacent turns; in the first state, the pulling wire extends out of the base through a current axial gap, winds around a free end of the artificial implant and then is mounted around a turn adjacent to the current axial gap.

Optionally, in the first state, the pulling wire extends out of the base through a current locking area, winds around a free end of the artificial implant and then returns to the current locking area or an adjacent locking area.

Optionally, the artificial implant has an axial direction, one end of the artificial implant in the axial direction is configured as a wire control end, the wire control end defines a hole for extending of the pulling wire, and according to a degree of deformation, the artificial implant includes:
a folding state, wherein the wire control end is radially folded and close to the base;
an expanding state, wherein the wire control end expands radially and away from the base;
wherein a round-trip path of the same pulling wire around the artificial implant does not repeated.

Optionally, there is a plurality of holes arranged at intervals, and in the expanding state, the same pulling wire extends through at least two holes.

Optionally, the round-trip path of the same pulling wire around the artificial implant is substantially triangular-shaped. Optionally, a number of the holes is twice the number of the pulling wires, and in the expanding state, each pulling wire corresponds to two holes.

The present application further provides a control mechanism having a distal end and a proximal end being opposite to each other and an axial direction extending between the distal end and the proximal end, including:
intervention components, including a first intervention component and a second intervention component;
a transmission member, a distal end if the transmission member being fixedly connected to the first intervention component;
a control handle, being connected to and controlling a proximal end of the transmission member; and
a linkage structure, including two mating portions being selectively linked, one of which is fixed to the second intervention component and the other is fixed to the transmission member, and a linkage state of the two mating portions being switched during the movement of the transmission member.

Optionally, in the axial direction, the first intervention component is located at a side of the second intervention component (may be the distal end or the proximal end). Optionally, there is a relative position wherein the first intervention component and the second intervention component are partly nested with each other. Optionally, the first intervention component and the second intervention component each include:
an inner shaft assembly, configured for carrying an artificial heart valve before release;
a sheath tube, being capable of wrapping around the artificial heart valve before release;
a base being connected to the artificial heart valve before release and axially limited to each other;
a locking member configured for limit the artificial heart valve before release in the base; and
a pulling wire connected to the artificial heart valve, configured for controlling the release process of the artificial heart valve.

Optionally, the control mechanism further includes a balloon body for carrying the artificial heart valve and capable of expanding the artificial heart valve.

Optionally, the two mating portions are linked or unlinked when the transmission member rotates.

Optionally, the two mating portions are linked or unlinked when the transmission member slides along the axial direction.

Optionally, the two mating portions drive the second intervention component to rotate or move linearly when they are linked.

The present application further provides a locking mechanism for connecting artificial implant with delivery system, including:
a first shaft, wherein a loading section is fixed to a distal end of the first shaft and opens towards a proximal end, configured for accommodating a distal portion of the artificial implant;
a pulling wire configured with a free end being capable of passing through and winding around the artificial implant or of being detached therefrom;
a third shaft being slidably mounted around the first shaft;
a locking assembly, wherein the artificial implant is bound to the locking assembly by the pulling wire during loading, the locking assembly including a locking member and a base fixed to the third shaft, the locking member and the base having:
a locking state in which they cooperate with each other to restrict the detachment of the pulling wire from the artificial implant; and
an unlocking state in which the cooperation is released to allow the pulling wire to detach from the artificial implant; and
a linkage assembly, acting between the first shaft and the locking member to selectively link the first shaft and the locking member; wherein
in the unlocking state, the locking member is rotatably mounted around the first shaft; and in the locking state, the locking member is linked with the first shaft and rotates along with the first shaft until it is combined with the base and binds the pulling wire.

Optionally, the linkage assembly includes two mating portions, one of which is connected to the loading section and the other is connected to the locking member, and axial movement of the loading section cause the two mating portions to be linked or unlinked.

Optionally, the two mating portions are configured as a linkage key and a linkage groove which are capable of being separated by axially sliding, and the linkage key is rotatably linked with the linkage groove when it is inserted into the linkage groove.

Optionally, there is a plurality of linkage keys connected to each other through a tubular component, and the plurality of linkage keys are arranged along a circumferential direction of the tubular component. Optionally, there is a plurality of linkage grooves connected to each other through a tubular component, and the plurality of linkage grooves are arranged along a circumferential direction of the tubular component. Optionally, at least a part of the locking member is configured as a connecting portion, and the linkage key or the linkage groove is set at a proximal end or a distal end of the connecting portion. Optionally, the connecting portion is located inside or outside the base.

Optionally, the connecting portion is configured as a tubular structure, the linkage groove is opened on a pipe wall of the connecting portion, and the linkage key is provided on an inner circumferential wall of the loading section. Optionally, an open side of the linkage groove is configured with an expansion structure.

Optionally, an inner circumference of the loading section is provided with a support member which is fixed to a distal end of the first shaft, and one of the two mating portions is provided on the support member.

Optionally, a limit structure is provided between the support member and the loading section to restrict their axial separation.

Optionally, the limit structure includes a positioning piece protruding radially from an outer circumference of the support member, and a positioning groove set on the inner circumferential wall of the loading section to cooperate with the positioning piece.

Optionally, the support member is a cylindrical structure, and the outer circumference of the support member is partially flipped to form the positioning piece.

Optionally, the support member included an outer cylinder connected to the loading section and an inner cylinder configured with the mating portion and fixed to the first shaft. Optionally, the outer cylinder and the inner cylinder are formed separately.

The present application further provides a catheter assembly for facilitating the withdrawal of artificial implant, including:
an intermediate shaft assembly, configured for connecting the artificial implant in a releasable manner through wire control;
a first shaft, extending through an interior of the intermediate shaft assembly to a distal end, and an extending portion of the first shaft being provided with a loading section which is tubular-shaped and open towards a proximal end, the loading section being configured as a radially deformable structure to adapt to being wrapped and folded by the artificial implant when the artificial implant is withdrawn; and
a catheter sheath, being slidably set on an outer circumference of the intermediate shaft assembly for cooperating with the loading section to receive the artificial implant during interventional delivery, and for receiving and wrapping the loading section when the artificial implant is withdrawn.

Optionally, the loading section is provided with a developing label.

Optionally, the loading section includes a composite multi-layer structure, and the developing label is located within an interlayer of the multi-layer structure.

Optionally, the developing label itself or in combination with the molding material is pre-formed into a strip structure and placed inside the interlayer.

Optionally, the developing label is made of powdered material and pre-mixed into the raw materials of the loading section.

Optionally, the loading section is configured with a plurality of pre-setting portions arranged along the circumference to guide its own radially deformation.

Optionally, the pre-setting portion is a radially inward concave crease and/or thinning area.

Optionally, the material of the loading section is elastic polymer materials such as PeBax, TPU, nylon, etc.

Optionally, the intermediate shaft assembly includes:
a third shaft being configured as a hollow structure and connected to a base at its distal end, wherein the base is configured with a locking hole that matches the locking member, the first shaft movably extends out of the distal end of the third shaft, and the locking member is set on the first shaft;
a second shaft movably mounted around the third shaft;
a pulling wire connected to the second shaft and cooperated with the base and the locking member to control the artificial implant in a wire controlled manner.

The present application further provides a loading method for an artificial implant based on wire control, including:
providing the above control mechanism, in which a free end of a pulling wire passes through a proximal end of the artificial implant and then is combined with a locking member and is bound to the base by the locking member; wherein
the pulling wire has a control end opposite to the free end, and moving the control end makes a portion of the pulling wire exposed out of the base be shorten gradually to drive a proximal end of the artificial implant to deform radially and fold;
providing a sheath tube and making the sheath tune be around the artificial implant and move relative to the artificial implant from a proximal end to a distal end until the artificial implant are completely folded by the sheath tube.

Optionally, after the free end of the pulling wire passes through the artificial implant, the loading method further includes: the loading section moving towards the proximal end and combining with the locking member to prepare for driving the locking member to move to combine with the free end.

Optionally, before moving the artificial implant and the sheath tube relative to each other until the sheath tube completely wraps around the artificial implant, the loading method further includes: moving the loading section back to the distal end to exit the artificial implant.

Optionally, after the loading section moves back towards the distal end to exit the artificial implant, the distal end of the artificial implant is compressed, and then the loading section is driven towards the proximal end to wrap around the distal end of the artificial implant.

The present application further provides a pre-loading method for an artificial implant based on wire control, including:
providing the above control mechanism where a free end of a pulling wire passes through a proximal end of the artificial implant; wherein
the free end of the pulling wire is configured with a wire loop and two support points, a mounting section is formed between the two support points and placed in a locking area of the base, so that the pulling wire is around a movement path of the locking member;
driving the locking member to rotate along the movement path, pass through the wire loop and engage into a locking hole of the base, thereby securing the free end of the pulling wire.

Optionally, the pre-loading method further includes:
the pulling wire has a control end opposite to the free end, and moving the control end makes the pulling wire drive a proximal end of the artificial implant to deform radially and fold;
providing a sheath tube and making the sheath tune be around the artificial implant and move relative to the artificial implant from a proximal end to a distal end until the artificial implant are completely folded by the sheath tube.

### BENEFICIAL EFFECT

The control mechanism of this application can achieve the expansion, release, or withdrawal of the artificial implant in the body, and the control of the pulling wire is smoother.

### BRIEF DESCRIPTION OF THE DRAWINGS

Fig. 1a is a schematic diagram of a control mechanism according to an embodiment of the present application for limiting an artificial implant.
Fig. 1b is a schematic diagram showing that the pulling wire is detached from the locking member of the control mechanism of Fig. 1a.
Fig. 2a is a schematic diagram of a control mechanism according to an embodiment of the present application for limiting an artificial implant.
Fig. 2b schematic diagram showing that the pulling wire is detached from the locking member of the control mechanism of Fig. 2a.
Fig. 3 is a schematic diagram of a control mechanism according to embodiment of the present application being configured with three pulling wires.
Fig. 4 is a schematic diagram showing a connection between each pulling wire and the second shaft of a control mechanism according to embodiment of the present application.
Fig. 5 is a schematic diagram of a stent of a control mechanism according to embodiment of the present application.
Figs. 6a to 6c are schematic diagrams showing the wire control end of the artificial implant of the present application being folded gradually.
Figs. 7a and 7b are schematic diagrams showing the artificial implant of the present application during threading and folding, respectively.
Fig. 8 is a schematic diagram showing the existing control mechanism that restricts the artificial implant by pulling wire.
Fig. 9 is a schematic diagram showing that the pulling wire in Fig. 8 is detached from the base to release the restriction on the artificial implant.
Fig. 10 is a schematic diagram of a conveying system according to an embodiment of the present application.
Fig. 11 is an enlarged view of part A in Fig. 10.
Fig. 12 is a schematic diagram showing the mating state between the base, locking member, and pulling wire of an embodiment of the present application.
Fig. 13a is an exploded view of the locking member, base, and pulling wire in Fig. 12.
Fig. 13b is a partial structural view of the distal side of the second shaft in Fig. 13a.
Fig. 14 is a left side view of the conveying system in Fig. 10.
Fig. 15 is a partial sectional view of the base along direction of B-B in Fig. 14.
Fig. 16a is a schematic diagram of a pulling wire at the free end according to an embodiment of the present application.
Fig. 16b is a schematic diagram of a pulling wire at the free end according to another embodiment of the present application.
Fig. 17 is a schematic diagram of a base according to an embodiment of the present application.
Figs. 18a-19 are schematic diagrams showing the pulling wire sequentially passing through the locking member of a control mechanism of the present application.
Fig. 20a is a schematic diagram of the pulling wire in the first state according to an embodiment of the present application.
Fig. 20b is a schematic diagram of the pulling wire in the second state according to an embodiment of the present application.
Figs. 21a-21c are schematic diagrams showing the pulling wires are sequentially combined with the locking member according to an embodiment.
Fig. 22 is a front view of a base according to an embodiment of the present application.
Fig. 23a is a schematic diagram of the positioning portion of the locking member according to one embodiment of the present application.
Fig. 23b is a schematic diagram of the positioning portion of the locking member according to another embodiment of the present application.
Fig. 24a is a schematic diagram showing the mating of the locking member and the base according to an embodiment of the present application.
Fig. 24b is a schematic diagram of the locking member in Fig. 24a.
Fig. 24c is a schematic diagram of the locking member in Fig. 24a after relative separation.
Fig. 25a is a schematic diagram showing the mating of the locking member and the base according to another embodiment of the present application.
Fig. 25b is an exploded view of Fig. 25a.
Fig. 26 is a schematic diagram of the threading of artificial implants in the prior art.
Fig. 27a shows a folding route of the artificial implant in the prior art.
Fig. 27b is a schematic diagram of the artificial implant in the prior art after being folded.
Fig. 28 shows a folding route of the artificial implant of the present application.
Fig. 29 is a front view showing the proximal end side of the artificial implant is pulled by the pulling wire in the control mechanism of an embodiment of the present application to fold radially.
Fig. 30a is a schematic diagram showing that the loading section is withdrawn and matches with the locking member of a control mechanism according to an embodiment of the present application.
Fig. 30b is a front view of the control mechanism in Fig. 30a (with the wire being removed for ease of observation).
Fig. 31 is a semi-sectional view of a control mechanism according to an embodiment of the present application, wherein the loading section and locking member are matched.
Fig. 32 is a schematic view of the loading section of a control mechanism according to an embodiment of the present application.
Fig. 33 is an exploded view of the loading section in Fig. 32.
Fig. 34a is an exploded view of the support member in Fig. 33.
Fig. 34b is a semi-sectional view of the support member.
Fig. 35 is a sectional view of a control mechanism according to an embodiment of the present application, wherein the loading section is away from the base.
Fig. 36a is a partial schematic diagram of a control mechanism according to an embodiment of the present application when the artificial implant is loaded on the loading section.
Fig. 36b is a schematic diagram showing the radial relationship between the loading section and the arm portions of the artificial implant of a control mechanism according to another embodiment of the present application.
Fig. 36c is a schematic diagram showing the radial relationship between the loading section, artificial implant, and catheter sheath of a control mechanism according to an embodiment of the present application.
Fig. 37 is a schematic view of an artificial implant according to an embodiment of the present application (leaflets, skirts, etc. are omitted).
Fig. 38 is a front view of an artificial implant according to an embodiment of the present application (leaflets, skirts, etc. are omitted).
Fig. 39 is a schematic view of a conveying system according to an embodiment of the present application.
Fig. 40 is a schematic view of a catheter sheath according to an embodiment of the present application.
Fig. 41a is a schematic view showing the control mechanism wrapping and restraining artificial implant of an embodiment of the present application.
Fig. 41b is a schematic diagram showing that the catheter sheath is driven to release the arm portions in the control mechanism of an embodiment of the present application.
Figs. 41c to 41e are schematic diagrams showing that the pulling wire is released by the control mechanism of the present application releasing to gradually expand the proximal end of the artificial implant.
Fig. 41f is a schematic diagram showing the control mechanism of the present application, in which two mating portions cooperate with each other to release the pulling wire.
Fig. 41g is a schematic diagram showing the control mechanism of the present application being withdrawn from the body.
Fig. 42 is a schematic diagram of a control handle according to an embodiment of the present application.
Fig. 43 is an exploded view of the push mechanism in Fig. 42.
Fig. 44 is a schematic diagram showing the first level of the telescope assembly in Fig. 42 extending to but the fixed seat.
Fig. 45 is a schematic diagram showing the telescopic assembly in Fig. 44 being driven by the push drive mechanism driving to move towards the distal end.
Fig. 46 is a schematic view of a control handle according to an embodiment of the present application.
Fig. 47 is an exploded view of Fig. 46.
Fig. 48 is a schematic diagram of the base of the control mechanism of the present application.
Fig. 49 is a semi-sectional view of the base part of the control mechanism of the present application.
Figs. 50a and 50b are schematic diagrams showing the locking member inserting and exiting the locking holes of the control mechanism of the present application.
Fig. 51 is a schematic diagram showing the locking member and base cooperating with each other of the control mechanism of the present application.
Fig. 52a is a schematic diagram showing the process of inserting the locking member into the locking hole of the control mechanism of the present application.
Fig. 52b is a partial schematic diagram of the base of the control mechanism of the present application.
Fig. 53 is a semi-sectional view of the wire control end of the base of the control mechanism of the present application.
Figs. 54a and 54b are schematic diagrams of the locking member of the control mechanism of the present application.
Fig. 55 is a schematic diagram of the extension path of the pulling wire in the locking member of the control mechanism of the present application.
Figs. 56a and 56b are schematic diagrams showing the linkage of a control mechanism according to an embodiment of the present application.
Figs. 57a and 57b are schematic diagrams showing the linkage of a control mechanism according to another embodiment of the present application.
Fig. 58 is a schematic diagram of the connecting portion of the locking member of the control mechanism of the present application.
Fig. 59 is a partial schematic diagram of the distal end of the first shaft of the control mechanism of the present application.
Figs. 60a to 60b are semi-sectional views showing the first shaft and the connecting portion being disengaged and engaged of the control mechanism of the present application.
Fig. 61 is a schematic diagram showing rotating of the first shaft and the connecting portion after being combined in the control mechanism of the present application.
Fig. 62 is a sectional view of the base of the control mechanism of the present application.
Fig. 63 is a schematic view of a control mechanism (the locking member combined with the pulling wire) of an embodiment of the present application.
Fig. 64 is a schematic view of the locking member and pulling wire in Fig. 63 when they are disengaged.
Fig. 65 is a schematic diagram of the assembly of the locking member, base, and pulling wire of an embodiment of the present application.
Fig. 66 is a schematic diagram of a conveying system according to an embodiment of the present application.
Fig. 67 is an enlarged view of part A in Fig. 66.
Fig. 68 is a partial schematic diagram of Fig. 67 after removing the artificial implant.
Fig. 69 is an enlarged view of the part C in Fig. 68 after being combined with the pulling wire.
Fig. 70 is an exploded view of the locking member and base of an embodiment of the present application.
Fig. 71 is a front view of a locking member and base according to an embodiment of the present application.
Fig. 72 is a partial cross-sectional view of along direction of E-E in Fig. 71.
Fig. 73 is an enlarged view of part F in Fig. 72.
Fig. 74 is a cross-sectional view taken along direction of D-D in Fig. 72.
Fig. 75 is a schematic diagram of the outer cylinder in Fig. 70.
Fig. 76 is a schematic diagram of the inner cylinder in Fig. 70.
Fig. 77 is a front view of the inner cylinder in Fig. 76.
Fig. 78 is a cross-sectional view of the inner cylinder taken along direction of G-G in Fig. 77.
Fig. 79 is a schematic diagram of a base according to another embodiment of the present application.
Fig. 80 is a schematic diagram of a base according to another embodiment of the present application.
Figs. 81 and 82 are schematic diagrams showing the locking member being combined with the pulling wire and sequentially passed through the locking hole.
Fig. 83 is a schematic diagram of the locking member and inner cylinder in the initial assembly stage of an embodiment of the present application (outer cylinder is removed for ease of observation).
Fig. 84 is a schematic diagram of Fig. 83 from another aspect.
Fig. 85 is a schematic diagram of the locking member and inner cylinder in Fig. 83 after being assembled.
Fig. 86 is a schematic diagram of the loading section and locking member of an embodiment of the present application being separated.
Fig. 87 is a schematic diagram of the loading section and locking member in Fig. 86 being combined.
Fig. 88 is a schematic diagram of the support member in the loading section of an embodiment of the present application.
Fig. 89 is a schematic diagram of the loading method and pre-loading method of an embodiment of the present application.

### DESCRIPTION OF THE EMBODIMENTS

The following will provide a clear and complete description of the technical solutions in the embodiments of the present application, in combination with the accompanying drawings. Obviously, the described embodiments are only a part of the embodiments of the present application, not all of them. Based on the embodiments of this application, all other embodiments obtained by those skilled in the art without creative labor are within the scope of protection of this application.

It should be noted that when a component is referred to as "connected" to another component, it may be connected to another component directly or there may be a middle component connected therebetween. When a component is considered to be "set on" another component, it may be set on another component directly or there may be a middle component set therebetween.

Unless otherwise defined, all technical and scientific terms used in this disclosure have the same meanings as those commonly understood by those skilled in the art belonging to the present application. The terms used in the disclosure of this application are only for the purpose of describing specific embodiments and are not intended to limit the scope of this application. The term "and/or" used in this disclosure includes any and all combinations of one or more related listed items.

In this application, the terms "first", "second", etc. are only used for descriptive purposes and should not be understood as indicating or implying relative importance or implying the quantity or order of the indicated technical features. Thus, the features limited to "first" and "second" may explicitly or implicitly include one or more of these features. In the description of this application, the meaning of "multiple/a plurality of" refers to at least two, such as two, three, etc., unless otherwise specified.

This disclosure describes an artificial implant and a delivery system for delivering the artificial implant into a human body. The delivery system includes a control handle and a catheter assembly, wherein the control handle may be connected to and control the catheter assembly which is used for performing interventional surgery. The catheter assembly includes a plurality of controlled components, and distal ends of the controlled components cooperate with each other to operate the artificial implant, such as releasing, withdrawing, locking position, adjusting spatial posture, etc. Each controlled component may be configured as a hollow tube, a solid rod, a flexible wire, or a combination of various forms. There are multiple controlled components, and at least two of them (taking the proximal end as an example) can slide relative to each other along the axial direction or rotate relative to each other about the axis. The force applying components on the control handle (i.e., the parts that the user directly operates and contacts), which are used to operate the controlled components, can be connected to the corresponding controlled component directly and fixedly for transmission, or, threads and rack-and-pinion may be used for transmission therebetween.

In the following description, many improvements regarding control handles or local structures can be implemented on the same control handle without obvious technical contradictions, but are not strictly limited to being implemented on the same control handle. For different numbers of controlled components and motion characteristics, or for control handles with further simplified structures, the embodiments may be implemented separately or combined appropriately.

The application site and structure of artificial implants are not strictly limited. Taking artificial heart valves as examples in some drawings or texts, the artificial heart valve generally includes a deformable stent and leaflets connected to the stent. The stent in whole is cylindrical-shaped, with a sidewall thereof being configured as a hollow mesh structure. Unless otherwise specified, the shape or size of the mesh structure is not strictly limited. An interior of the stent is configured as a blood flow channel, and multiple leaflets cooperate with each other to control the degree of opening and closing of the blood flow channel inside the stent. In order to locate within the body, positioning structures that can interact with surrounding native tissues, such as anchor spikes, arms, etc., may be provided around the stent. To prevent leakage, skirts or anti-leakage materials may be provided on the interior and/or exterior of the stent.

According to different expansion modes, the stent is processed using corresponding materials, such as nickel titanium alloy with shape memory and self-expanding ability, or stainless steel material for balloon expansion. The stent itself may be formed by cutting pipes or weaving wires, and the leaflets may be connected to the stent by sewing, bonding, or integral molding.

Taking the self-expanding stent as an example, its expansion and retraction may be controlled by a sheath catheter wrapped around the outer circumference of the stent. According to the different parts of the stent exposed to the sheath catheter, corresponding control may be carried out. The stent may also be controlled by a pulling wire, which passes through the structural gap (or wire hole) of the stent. By adjusting the tension of the pulling wire through the control handle, the degree of expansion of the stent can be changed. When the pulling wire is pulled away from the stent, the stent is allowed to be completely released. Of course, the control of the pulling wire is also achieved through various controlled components in the catheter assembly.

The stent of artificial implant generally has a connection structure that matches with the catheter assembly to limit positions relative to each other and prevent unnecessary positional deviation during delivery. The artificial implant is in a radially compressed state when delivery, that is, in a loaded state. After releasing the constraint of the catheter assembly and expanding radially in the human body, the stent is in an expanding state. Unless otherwise specified, the shape of the artificial implant is understood as the shape in the expanding state, without considering the local deformation caused by surrounding tissue pressure.

Due to the complexity of the internal of the human body, the catheter assembly often requires bending operations. Corresponding bending components may be configured as tubes or wires, with the distal end thereof acting on the bending member and the proximal end thereof adjusting the bending amplitude or direction through a control handle.

When used to indicate direction, the proximal end in the disclosure generally refers to the side adjacent to the operator (such as a doctor), and the distal end is the side relatively far away from the operator. Along the intervention path, each component has its own distal and proximal ends that are opposite to each other. In theory, when the catheter assembly and control handle are completely straightened, a straight line between the proximal end and the distal end determines the axial direction, and correspondingly determines the radial direction which is perpendicular to the axial direction and the circumferential direction which is around the axial direction. When used to refer to a structure, the term "end" in the disclosure refers to the endpoint, or a point or region in this direction, or a specific structure connected to that point or region, of the structure.

The present application provides a control mechanism for an artificial implant, including a base 10, a pulling wire 11, and a locking member 13, wherein the base 10 defines a locking hole, the pulling wire 11 includes a free end 111 that can pass through and wind around, or be detached from the artificial implant 60, and the locking member 13 in whole is located at the distal end of the base 10 and is rotatably engaged with the base 10 to lock the free end 111 of the pulling wire 11. The base 10 has a distal end 32 and a proximal end 31 that are opposite to each other, as well as an axial direction extending between the distal end 32 and the proximal end 31. Unless otherwise specified, the proximal end, distal end, and axial direction are also applipulling wire to other components of the control mechanism, as well as the control handle and the delivery system of the following embodiments. The free end 111 is the end where the pulling wire 11 first passes through and finally detaches from the artificial implant 60, and may also be understood as the farthest end when the pulling wire 11 is straightened. The other end of the pulling wire 11 opposite the free end 111 is the control end, which may be fixed to the base 10 directly or extended towards the proximal end for controllability.

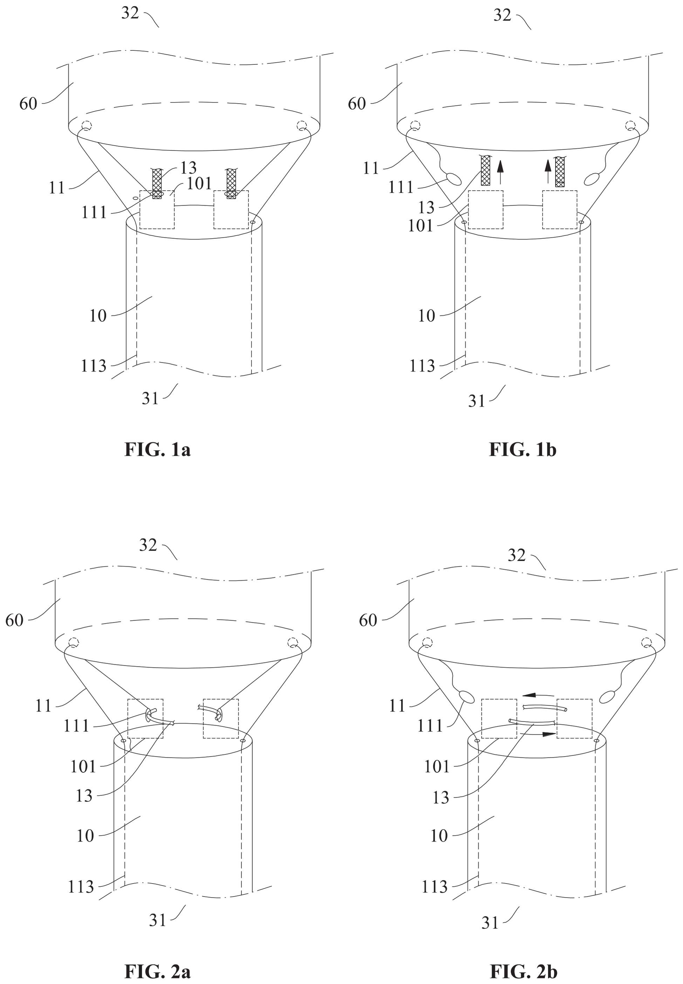

The free end 111 has a first state restricted to the base 10 (as shown in Fig. 1a) and a second state released from restriction (as shown in Fig. 1b). The locking member 13 is in active cooperation with the base 10, and the locking member 13 is in cooperation with the free end 111 of the pulling wire 11 to change the state of the free end 111. In the first state, the free end 111 is combined with the locking member 13 and the locking member 13 cooperates with the base 10 to restrict the pulling wire 11 from disengaging from the locking member 13. At this time, the free end 111 can be understood as being fixed to the locking member 13. In this state, the pulling wire 11 is always connected to the artificial implant 60, and the expansion process (i.e. expansion degree) of the artificial implant is adjusted based on the length of the pulling wire 11 exposed out of the control mechanism, as well as the expansion/folding speed of the artificial implant is controlled based on the rate of change of the pulling wire. When necessary, the pulling wire 11 can also be used for the withdrawal of artificial implants.

In the second state, the free end 111 disengages from the locking member 13. At this time, the free end 111 can be understood as being released, thereby releasing the connection between the artificial implant and the control mechanism. Subsequently, the control mechanism as a whole can be withdrawn out of the human body, leaving the artificial implant in the predetermined position inside the human body.

When the locking member, the base, and the pulling wire are pre-assembled out of the body, the extension path of the pulling wire 11 enters the proximal end of the base, and then extends out from the distal end of the base and wraps around the artificial implant before being combined with the locking member 13.

After releasing and detaching from the artificial implant, the control end is driven to move the pulling wire 11 towards the proximal end to withdrawal the pulling wire. When the artificial implant is withdrawn, the pulling wire 11 is in the first state, and the control end 113 is operated to reduce the length of the pulling wire 11exposed out of the control mechanism.

The locking member 13 is located at the distal end of the base 10, which is conducive to observation and operation during pre-assembly outside the body, thereby facilitating assembly. Especially, the locking member 13 and the base 10 are not nested radially, so as to form sufficient radial space for extending of the pulling wire and make the pulling wire move smoother. In addition, the maximum radial size of the base 10 and/or the locking member 13 can be correspondingly reduced, facilitating interventional delivery in the body. Of course, the control mechanism of this application and other embodiments including the control mechanism of this embodiment are also applipulling wire to simulation training of interventional delivery operations in the body. The interior of prostheses (such as artificial organs used for simulation training or observation) can also be understood as out of the body.

The control end 113 of the pulling wire 11 can extend to and be controlled by the control handle, and its movement, which specifically may be axial movement, rotation, or winding, can change the length of the pulling wire 11 exposed out of the base 10.

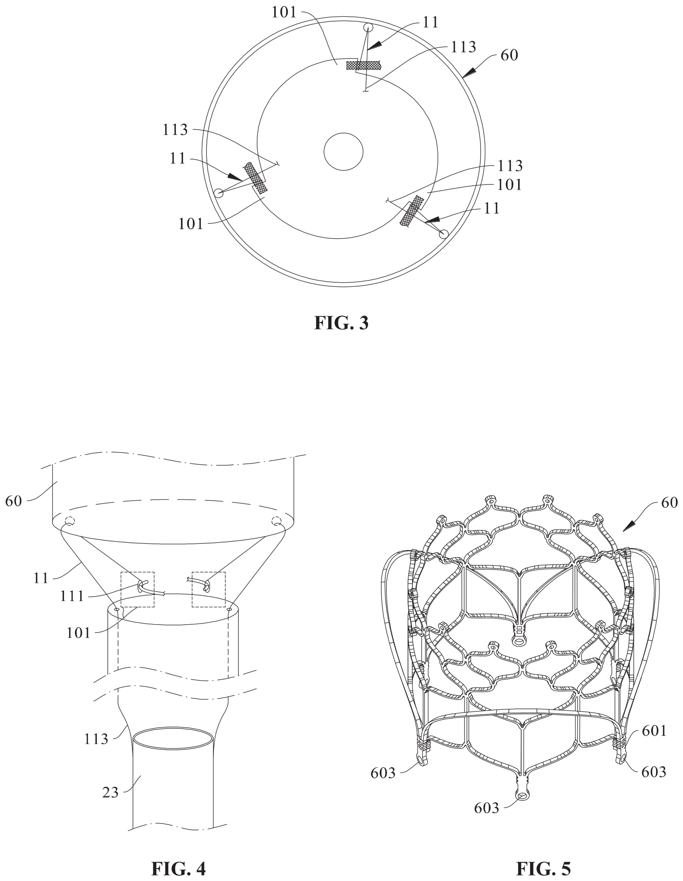

Among them, the artificial implant 60 is a cylindrical structure with corresponding circumferential direction, and there are multiple pulling wires 11. The control ends 113 of the pulling wires 11 can be controlled independently or move synchronously. The positions where the pulling wires 11 act on the artificial implant 60 are arranged at intervals along the circumferential direction of the artificial implant 60, which can improve the synchronization of the contraction and expansion of the artificial implant 60. For example, as shown in Fig. 3, there are three pulling wires 11, and there are three positions where the pulling wires 11 act on the artificial implant 60 arranged at intervals along the circumferential direction of the artificial implant.

Correspondingly, there are multiple locking areas 101. In the first state, the free end 111 of each pulling wire 11 corresponds to one of the multiple locking areas 101, thereby avoiding interference between the pulling wires 11. The multiple locking areas 101 may be separated through the structure of the base, or additional separation components may be provided on the base to separate the multiple locking areas 101.

The control end 113 of each pulling wire 11 may extend to the control handle directly, or an intermediate component may be adopted to realize indirect transmission, so as to reduce the risk of the multiple pulling wires 11 intertwining with each other. For example, as shown in Fig. 4, the control mechanism further includes a second shaft 23, and the control end 113 of each pulling wire 11 is connected to the second shaft 23. By operating the second shaft 23, the pulling wires 11 are synchronously controlled

As shown in Fig. 5, the artificial implant 60 has an axial direction, and one end of the artificial implant 60 in the axial direction is configured as a wire control end 601, which may be either the distal end or proximal end of the artificial implant 60. In this embodiment, the wire control end 601 is located at the proximal end of the artificial implant, and has a hole 603 for extending of the pulling wire (not shown in the drawings for clarity) therethrough.

The formation of hole 603 can be, for example, the artificial implant including a hollow area that form the hole, wherein the hollow portion is one of the structural gaps in the artificial implant; or, drill holes directly into the artificial implant to form the hole (as shown in Fig. 5); or, component with the hole being configured independently relative to the artificial implants (such as connected to artificial implant through welding, etc.).

Among them, there are multiple holes 603 spaced from each other. In the expanding state, the same pulling wire 11 passes through at least two of the multiple holes 603.

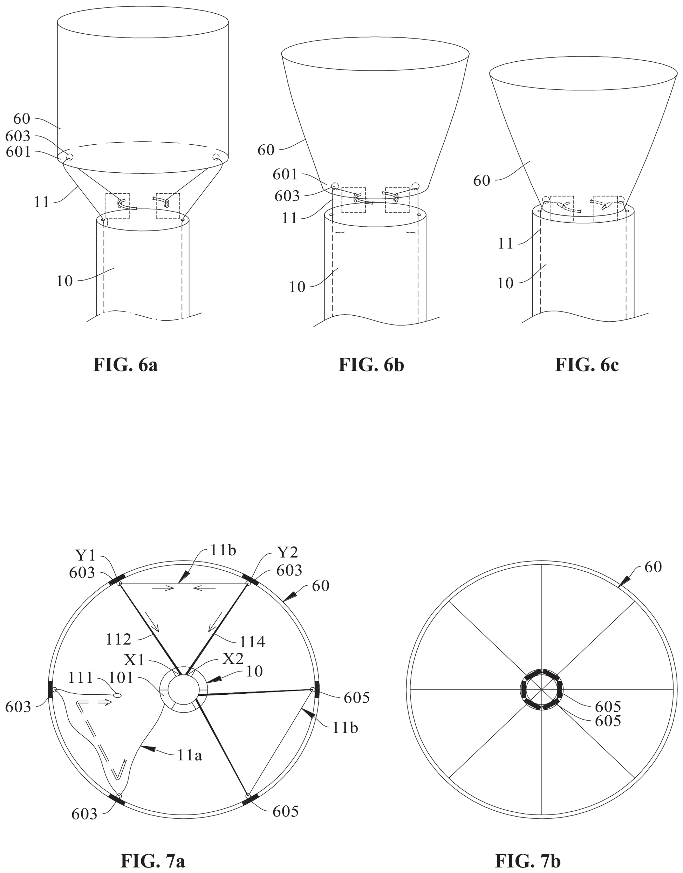

In the first state, the free end 111 of the pulling wire can limit the complete separation of the artificial implant 60 from the control mechanism, but the artificial implant can be allowed to deform to a certain extent by adjusting the length of the pulling wire exposed out of the base, which may be understood as changing the position of the artificial implant 60 relative to the base. Therefore, when the free end 111 of the pulling wire is maintained in the first state, the artificial implant 60 can still have multiple states according to the degree of its deformation, such as have relative states of:
an expanding state, wherein the wire control end 601 expands radially away from the base 10 (as shown in Fig. 6a), and the round-trip path of the same pulling wire 11 around the artificial implant 60 does not coincide;
a folding state, wherein the wire control end 601 is radially folded close to the base 10 (as shown in Fig. 6c); and
an intermediate state, wherein the wire control terminal 601 is in a state between the expanding state and folding state (as shown in Fig. 6b).

Among them, as shown in Figs. 5 to 7a, the multiple holes 603 are arranged at intervals along the circumferential direction. In the expanding state, the same pulling wire **11** passes through at least two holes 603, that is, one pulling wire 11 can act on two circumferential positions of the wire control end 601. In a preferred embodiment, the number of the holes 603 is twice the number of the pulling wires 11, and each pulling wire 11 corresponds to two holes 603. This enables synchronous withdrawal and expansion of the wire control end 601 of the artificial implant 60 in the circumferential direction, reduces the number of the pulling wires, avoids entanglement and friction at the proximal ends of the pulling wires, and correspondingly reduces the space for accommodating the pulling wires.

For the artificial implant 60 as a whole, at least two pulling wires should be configured. In addition, for the same hole, only one pulling wire is allowed to pass, so as avoid interference caused by reciprocating threading.

As shown in Fig. 7a, in this state, the extension direction (radial direction) of the tension pulling wire between the artificial implant 60 and the base 10, that is, the direction of traction force, is perpendicular to the movement direction (circumferential direction) for unlocking the locking member, which is more convenient for unlocking.

For example, as shown in Fig. 7a and Fig. 7b, the round-trip path of the same pulling wire 11 around the artificial implant generally encloses a triangular area, that is, the round-trip paths do not repeated. Otherwise, if the round-trip path is repeated, the extension path of the pulling wire 11 will be a single straight line or curve line, which will not enclose a certain area. The relationship between the extension path of the pulling wire 11 and the base 10 and locking member 13 will be described in the following embodiments.

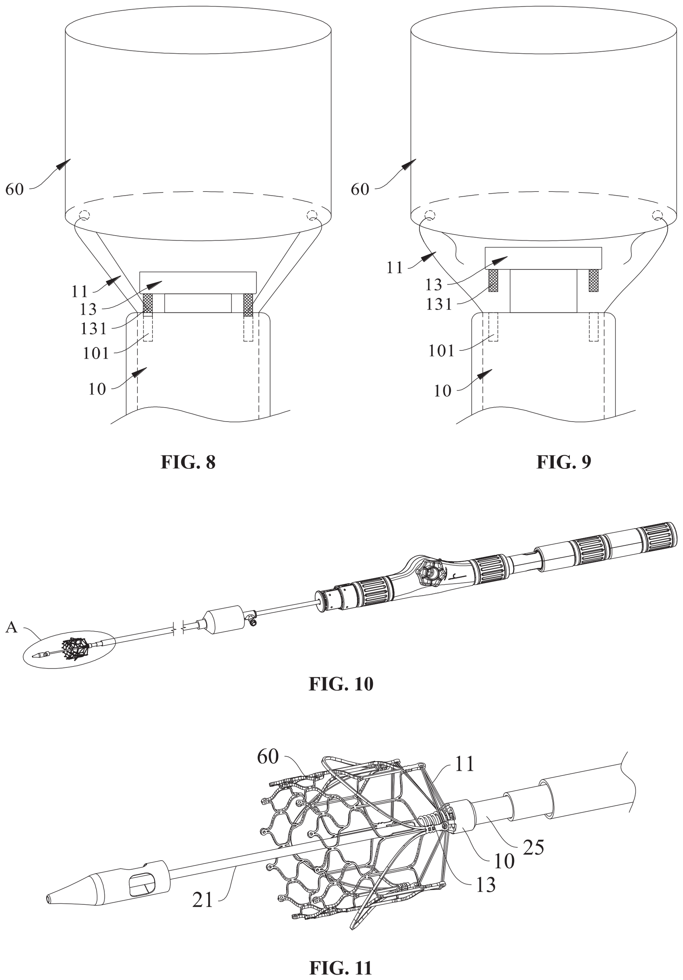

As shown in Figs. 10 to 15, the base 10 has a locking area 101, and the locking member 13 has a positioning portion 131 that enters or exits the locking area 101 during its rotation. The positioning portion 131 cooperates with the free end 111 of the pulling wire 11 to restrict the artificial implant 60. The interior of the base 10 defines a first cavity 103, the proximal end side of the base 10 defines a first opening 104 communicated with the first cavity 103, and the outer circumferential surface of the base 10 defines a second opening 108 communicated with the first cavity 103. The pulling wire 11 passes through the first cavity 103. One end (i.e., the control end) of the pulling wire 11 extends through the first opening 104 towards the proximal end and out of the base 10. The other end of the pulling wire 11, i.e., the free end 111, extends out of the base 10 through the second opening 108 to connect the artificial implant 60.

The pulling wire 11 passes through the first cavity 103 of the base 10, reducing the occupation of radial space. Moreover, the locking member 13 is not inside the first cavity 103, so that the movement of the pulling wire 11 will not be interfered by the locking member 13, making the movement of the pulling wire 11 smoother, and facilitating the expansion control of the artificial implant.

The locking area 101 may be located within the overall outer contour of the base 10, so as to accommodate the free end in the first state. In addition, the locking area 101 may be opened towards the distal end to optimize the extension path of the pulling wire 11. For example, the locking area 101 is located at the second opening.

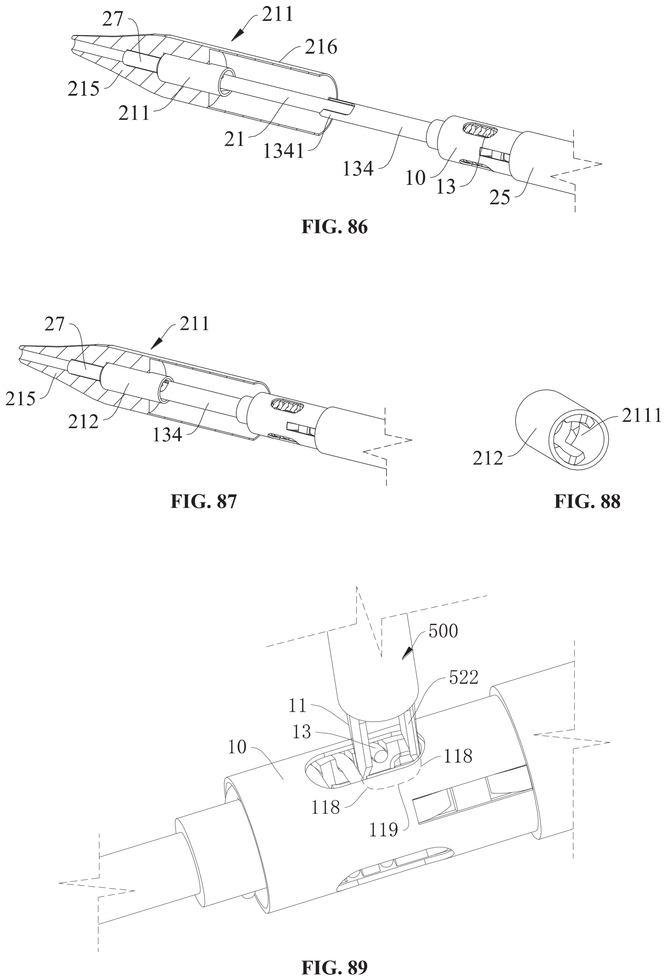

In conventional techniques, such as Figs. 8 and 89, it is shown that the locking member 13 slides axially to make the positioning portion 131 disengage from the locking area 101, thereby allowing the pulling wire 11 to detach from the artificial implant. In this embodiment, the positioning portion 131 serves as a part of the locking member 13, and there is displacement at least in the circumferential direction during operation, which can reduce or even eliminate the axial stroke variation of the locking member 13, reduce the axial space occupation of related components and even the proximal control handle, being more conducive to the configuration of the transmission mechanism.

In the first state, the pulling wire 11 is constrained by the artificial implant 60 and mainly undergoes radial movement. In the second state, the free end of the pulling wire 11 is free. Among them, the range of the second opening 108 includes the radial side wall and distal end wall of the base 10, which is conducive to the movement of the pulling wire 11 in all directions in the first state, as well as the quick and smooth withdrawal in the second state.

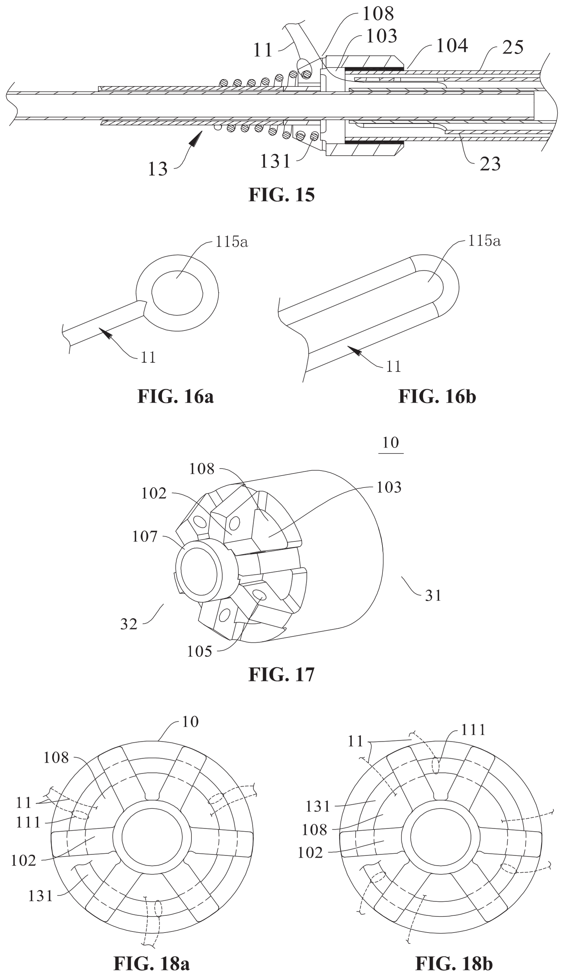

Regarding the cooperation between the free end 111 and the positioning portion 131, in one embodiment, the free end 111 has a ring 115a. In the first state of the free end 111, the positioning portion 131 penetrates the ring 115a; and in the second state of the free end 111, the positioning portion 131 exits the ring 115a. The ring 115a can be independently configured (as shown in Fig. 16a) or wound by the pulling wire 11 (as shown in Fig. 16b). The pulling wire 11 may be single strand or multiple strands. For example, the pulling wire 11 may be single strand extending to the control end 113, or, may be double strand extending in parallel to the control end 113, or, may be double strand extending in parallel for a period of time and then merging and extending to the control end 113.

Along the circumferential direction of the base 10, there are multiple locking areas 101 arranged at intervals, and the positioning portions 131 engages into or disengages from the locking areas 101 in sequence with the movement of the locking member 13.

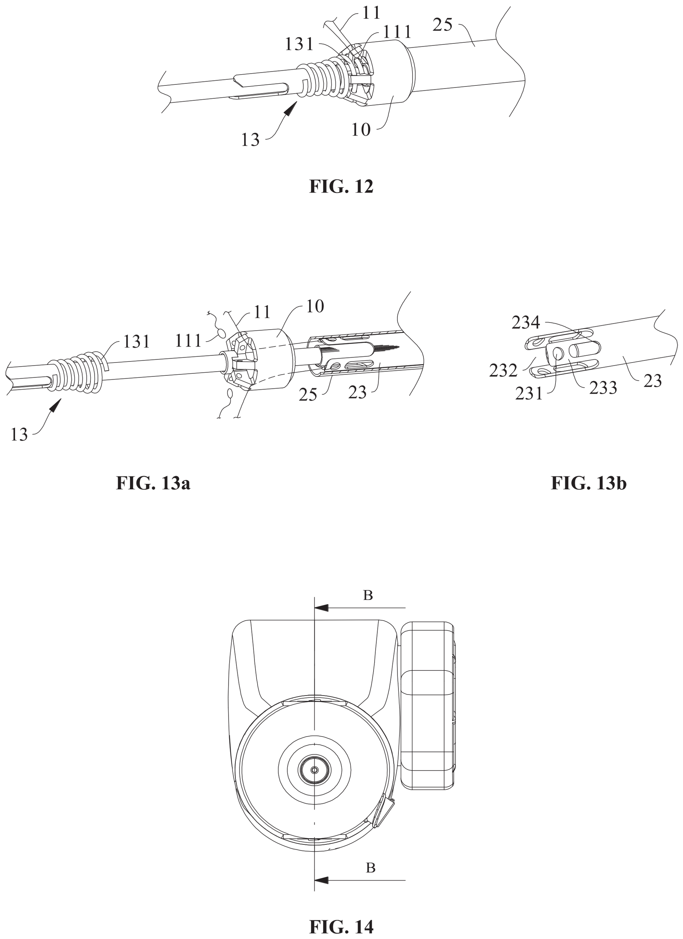

In one embodiment, as shown in Figs. 12 and 13a, the control mechanism further includes a third shaft 25 connected to the base 10 fixedly. The proximal end of the third shaft 25 extends and can be controlled by the control handle. The third shaft 25 can keep the base 10 relatively fixed in the circumferential direction, allowing the positioning portion 131 to rotate relative to the base 10. In the radial direction, the third shaft 25 is located around the second shaft 23. The third shaft 25 and the second shaft 23 are both pipe fittings, wherein the distal end of the second shaft 23 is configured with connecting holes 231 for winding and connecting the control ends of the pulling wires. The connecting holes 231 correspond one-to-one with the pulling wires, including the mutual correspondence in quantity and position. In the drawings, there are three connecting holes 231 arranged at intervals along the circumferential direction.

As shown in Figs. 13a and 13b, in one embodiment, multiple notches 232 arranged at intervals are defined in a wall of the distal end the second shaft 23, and a connecting arm 233 is formed between two neighboring notches 232. The connecting holes 231 are defined at the distal end of the connecting arms 233. During the movement of the second shaft 23 towards the distal end or proximal end, the connecting arms 233 adapt to the movement of the pulling wires and deform, including at least radial deformation. In one embodiment, a deformable hole 234 is defined in a portion of the connecting arm 233 which is located at the proximal end side of the connecting hole 231.

As shown in Figs. 17-18b, in one embodiment, the base 10 is configured with multiple separating members in the circumferential direction, and two adjacent separating members define the second opening 108. Therefore, there are multiple second openings 108 arranged at intervals along the circumferential direction of the base 10. The separating members are configured as the ribs 102 shown in the drawings. The strip-shaped structure minimizes its own circumferential span as much as possible, maximizing the circumferential span of the second opening and facilitating the arrangement of the pulling wire. For threading method, for example, one pulling wire extends through a locking area and then returns to this locking area; or, one pulling wire extends through a locking area and then returns to an adjacent locking area, this will allow the two action points to be sufficiently close. At the same time, the ribs 102 also serve as reinforcing ribs to maintain the basic strength requirements of the base.

In the first state, the free end 111 extends out of the base 10 through the corresponding second opening 108, winds around the artificial implant, and returns to the locking area corresponding to the same second opening 108 (as shown in Fig. 18a), or returns to a position adjacent to the second opening 108 in the circumferential direction (as shown in Fig. 18b).

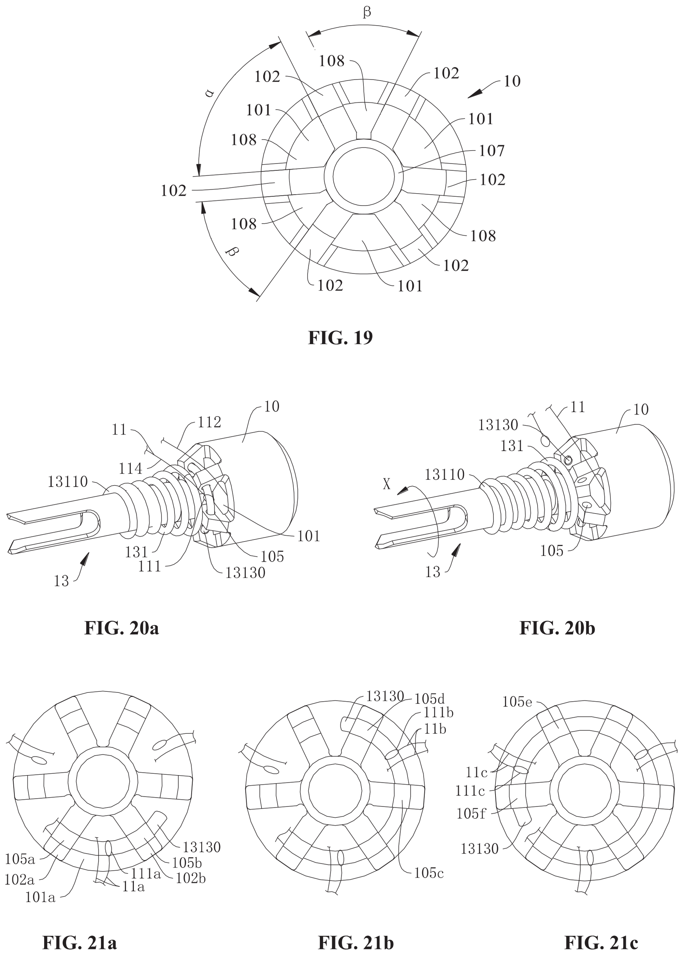

As shown in Fig. 19, the position adjacent to the second opening 108 in the circumferential direction may be another adjacent locking area 101, or a channel separately configured for extending of the pulling wire. The formation of this channel can be based the same principle as the locking area in structure. For example, there are six second openings 108, wherein three of them spaced apart from each other serve as channels for the free ends of the pulling wires to extend out of the base 10, and the other three of them spaced apart from each other serve as locking areas 101. For example, as shown in Fig. 19, in the circumferential direction, the span of the locking area 101 is α, and the span of the channel adjacent to the locking area 101 is β, wherein α/β = 1.1~2.

As shown in Fig. 17, all of the ribs 102 are converged and fixed to each other at the distal end of the base 10 to form an annular structure 107. All of the ribs 102 are continuously distributed in the circumferential direction at the proximal end 32 of the base 10 to form a cylindrical structure. An inner cavity of the cylindrical structure is part of the first cavity 103. An inner hole of the annular structure 107 can be used for other pipe fittings, such as the first shaft described below, to pass through.

In one embodiment, as shown in Figs. 20a and 20b, the base 10 is provided with a locking hole 105 for inserting the positioning portion 131. The positioning portion 131 includes:
a locking state, wherein the positioning portion 131 is inserted into the locking hole 105, restricting the free end 111 of the pulling wire 11 from disengaging (as shown in Fig. 20a); and
an unlocking state, wherein the positioning portion 131 exits the locking hole 105, allowing the free end 111 of the pulling wire 11 to disengage (as shown in Fig. 20b).

As shown in Fig. 21a, the locking area 101a is between two ribs 102a and 102b. In the first state, the free end 111 of the pulling wire 11 is located in the current locking area 101. Locking holes 105a and 105b are correspondingly defined in opposite sides of the two ribs102a and 102b. Of course, the locking holes may pass through the ribs where they are located. One section of the positioning portion 131 is located in the locking area 101a, and two ends of this section are inserted into the locking holes 105a and 105b, respectively. Combined with the moving direction of the locking member 13, an open side of the locking hole 105 is oriented in the circumferential direction of base 10.

When the positioning portion 131 moves in a first direction (as shown by X in Fig. 20b), the free end 111 switches from the second state to the first state. The locking hole 105 extends through the rib 102 along the first direction, and has forward and backward openings opposite to each other, wherein the forward opening faces the first direction.

In terms of quantity, there are multiple ribs 102 configured with the locking holes 105. The positioning portion 131 as a whole has a tail end 13110 and a head end 13130 opposite to each other. The head end 13130 is inserted into or exits from the locking holes 105 in sequence with the rotation of the locking member 13 (as shown in Fig. 20a). Taking the insertion of the positioning portion in sequence as an example, as shown in Figs. 21a-21c, the head end 13130 is sequentially combined with the locking hole 105a, the free end 111b, the locking hole 105d, the locking hole 105e, the free end 111c, and the locking hole 105f. The order of detachment is opposite to the order of combination. The advantage of sequential insertion is reflected in the sequential combination between the positioning portion and the pulling wire during external pre-assembly, which facilitates assembly.

In a preferred embodiment, all of the ribs 102 are provided with locking holes 105 for the positioning portion 131 to sequentially rotate through and provide certain motion guidance. During the process of rotating and inserting the locking member into the locking holes, the head end 13130 finally abuts against the side wall of one of the ribs 102 or is inserted into the corresponding locking hole, playing a limiting role.

When the positioning portion 131 is configured with a helical shape in multiple turns, the positions of the locking holes 105 on the ribs 102 are adapted to the positioning portions 131. Among them, there are one or multiple locking holes 105 on the same rib 102, wherein the multiple locking holes 105 are arranged in sequence along the extension direction of the rib 102 where they are located.

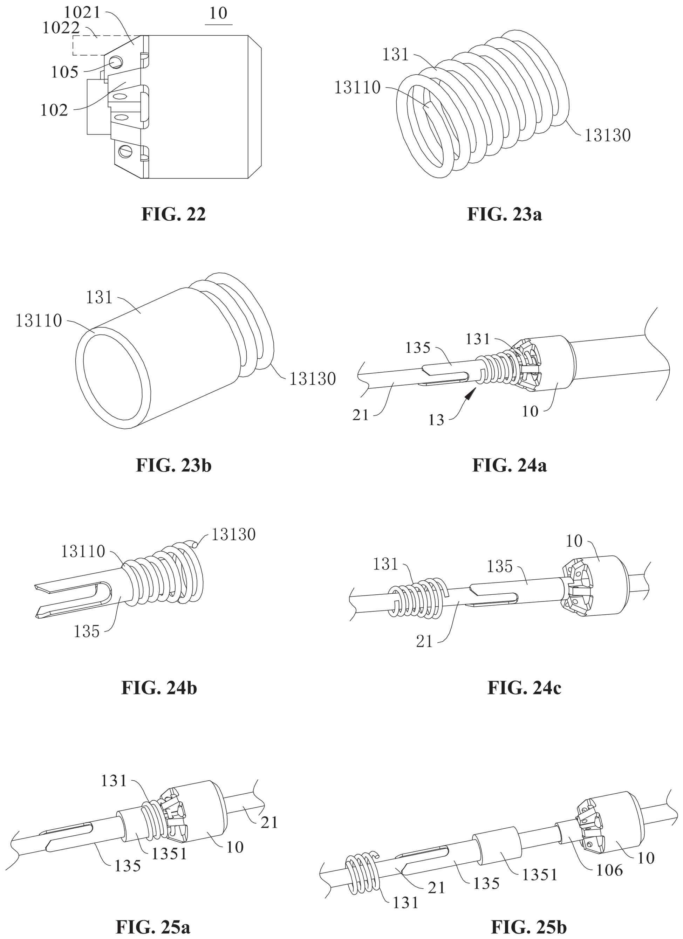

Referring to Fig. 22, in one embodiment, one section of the rib 102 along the axial direction is a bending section 1021 extending radially and inwardly, and the locking hole 105 is located at the bending section 1021 of the corresponding rib 102. All of the bending sections 1021 as a whole form an avoidance zone 1022 (as shown by the dashed area in the drawings), and the proximal end of the artificial implant in the folding state is located within the avoidance zone 1022. It is convenient to guide the loading section described below to be partially placed in the avoidance zone 1022 and aligned with the outer circumference of the base 10 when retracting.

Based on the rotation characteristics of the locking member and the circumferential distribution of the pulling wires, the positioning portion 131 has a curved shape and the curved extension path is around the base. The curved configuration can fully utilize the circumferential space to maintain the necessary length of the locking member, realizing the control of the free ends of all pulling wires through one locking member. It also eliminates the axial dimension changes of the control mechanism, retains the original function of the locking member fitting with the pulling wires one by one, facilitating the assembly of the locking member, the pulling wire, and the base during the assembly process.

Specifically, the open side of the locking hole faces the circumferential direction of the base, and the positioning portion is rod-shaped. The rod-shaped positioning portion may be a straight rod or a curved rod, with a quantity of one and cooperating with the free ends of all of the pulling wires. The positioning portion is engaged into or disengaged from the locking hole with the rotation of the locking member. For example, as shown in Figs. 23a and 23b, the positioning portion 131 is configured as a helical structure, which is wound at least once. For example, the helical structure is a multi-turns structure, with the multiple turns arranged along the axial direction (each turn extends around the base). The generatrix of the helical structure may be a diagonal line (adjacent to or away from the axis of the base) or a straight line, for example, the helical extension path is a cylindrical helical or at least partially a conical helical.

As shown in Figs. 24a-24c, the locking member 13 has a connecting portion 135 located at the distal end of the base 10, and the control mechanism further includes a first shaft 21 that cooperates with the connecting portion 135 in transmission, so as to drive the locking member 13 to rotate relative to the base 10. The proximal end of the first shaft 21 is connected to and controlled by the control handle.

Among them, along the winding direction of the helical structure, the positioning portion 131 as a whole has a tail end 13110 and a head end 13130 that are opposite to each other, with one end being fixed to the connecting portion 135 and the other end being free. The connection method between the positioning portion 131 and the connecting portion 135 includes separately forming and then connecting by, for example, welding or assembly, or integrally forming.

In one embodiment, a positioning mechanism is provided between the connecting portion 135 and the base 10 for maintaining axial positions of the connecting portion 135 and the base 10 during pre-assembling. The connecting portion 135 may be tubular-shaped with constant diameter. The positioning mechanism includes a first end surface located at the proximal end of the connecting portion and a second end surface located at the distal end of the base, wherein the second end surface is in contact with the first end surface. During pre-assembly, the first end surface abuts against the second end surface, and the positioning portion is in place with the locking hole of the base.

Among them, the head end 13130 of the positioning portion 131 needs to adapt to the radial position of the locking hole, and a part of the head end 13130 near the tail end 13110 needs to be connected to the connecting portion 135 fixedly. The outer diameter of the connecting portion has a significant deviation from the radial position of the locking hole, so that the helical extension path of the positioning portion 131 is a conical helical. Alternatively, as shown in Figs. 25a-25b, the proximal end of the connecting portion 135 expands radially and outwardly to form a connecting section 1351, the distal end of the base 10 has an extension section 106, and the connecting section 1351 is nested with the extension section 106. In the drawings, the connecting section 1351 is a cylindrical structure and mounted around the extension section 106. The connecting section 1351 compensates for the aforementioned deviation, so that the helical extension path of the positioning portion 131 is a cylindrical helical. At this time, a bottom wall of the connecting section 1351 serves as the first end surface.

The connecting portion 135 is movably mounted around the outer circumference of the distal end of the first shaft 21, and the connecting portion 135 can be axially separated from the distal end of the first shaft 21. When the connecting portion 135 and the first shaft 21 are matched, the connecting portion 135 and the first shaft 21 can be synchronized in the circumferential direction, and at least the proximal end of the first shaft 21 can be controlled to drive the locking member 13 to rotate. As for axial separation, it means that the connecting portion 135 and the first shaft 21 can be separated from each other to release their cooperation or combined with each other to maintain synchronization, and the specific cooperation relationship between the connecting portion 135 and the first shaft 21 can be switched according to operational requirements.

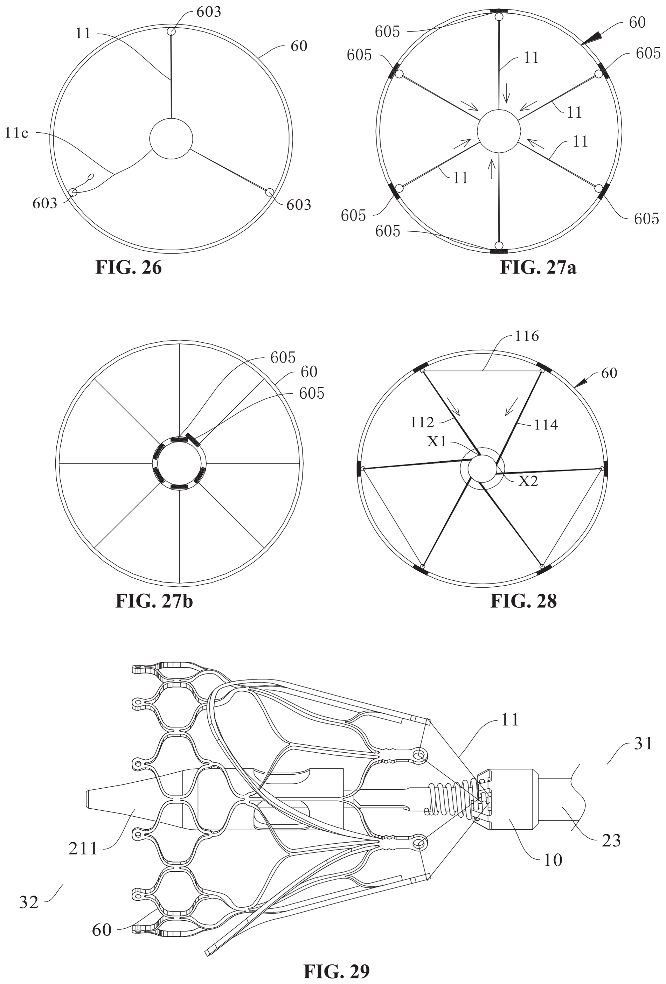

As shown in Fig. 28, regarding the extension path of the pulling wire 11, in the first state, the pulling wire 11 has a forward section 112, a return section 114, and an intermediate section 116 between the forward section 112 and the return section 114, cooperatively forming the triangular-shaped round-trip path in the above embodiment. The free end 111 is set at the distal end of the return section 114. In one embodiment, the positioning portion is a helical structure with multiple turns, and there is an axial gap between adjacent turns. The forward section can pass through the axial gap at the proximal end of the positioning portion and interact with it to form a first action point (X1), and the return section is connected to the positioning portion and interacts with it to form a second action point (X2). The two action points are close to each other, making the movement of the artificial implant at different positions more synchronized during withdrawal and expansion.

In another embodiment, as shown in Fig. 20a, the forward section 112 extends out through the locking area 101 (without through the axial gap) directly and interacts with it to form the first action point (X1), and the return section 114 is connected to the positioning portion 131 and interacts with it to form the second action point (X2). Based on the above, the space of the locking area 101 is larger than the axial gap, which facilitates the extending of the pulling wire. During pre-assembly, the pulling wire can extend through the base first, and then the locking member can be rotatably engaged with the base, and at the same time the free end can be inserted for easy assembly. The pulling wire 11b is in a state after threading, and the bolded parts are the forward section 112 and the return section 114 of the pulling wire 11b. As shown in Fig. 7a, the pulling wire 11b forms two action points (X1, X2) in the locking area 101 or the positioning portion 131, as well as two action points (Y1, Y2) between it and the artificial implant.

Here is a description based on existing technology: as shown in Fig. 26, there are three holes 603 arranged at intervals in the circumferential direction of the artificial implant 60, and correspondingly, there are three pulling wires. The specific extension path refers to pulling wire 11c, and its round-trip path is repeated. During the folding process, the force generated by the pulling wire is only in the radial direction, while the artificial implant will generate corresponding stress in the circumferential direction and react on the pulling wire, gradually increasing the force required to be applied to the pulling wire and affecting the operating feel.

However, in this embodiment, the pulling wire is configured as a triangle, which not only generates a radial force, but also generates a force along the connecting line of the two action points (Y1, Y2) (as shown by the solid arrows in Fig. 7a) to directly overcome the stress changes during the deformation process of the artificial implant. The overall force change of the pulling wire is smaller than that of the existing wire, and the operation feel is more delicate.

In conventional technology, the spacing between adjacent two holes 603 is relatively wide (i.e., the circumferential span is large). The holes 603 are opened on the connecting ears 605 (as shown in Fig. 27a), and the adjacent connecting ears 605 are independently connected with pulling wires. During the folding process, the adjacent connecting ears 605 may eventually be misaligned and stacked with each other (as shown in the bolded part of Fig. 27a), which affects subsequent expansion. However, in this embodiment, the extension path of the pulling wire makes the connecting ear 605 and the structural gap be mostly or completely controlled by the pulling wires, reducing or even eliminating the problem of misalignment and stacking during folding (as shown in Fig. 7b).

The two action points (X1, X2) are in the same locking area and adjacent to each other, and the round-trip path of the pulling wire roughly forms an equilateral triangle, that is, the lengths of the bolded forward section 112 and return section 114 in Fig. 7a are equal, and correspondingly the movement speeds of the forward section 112 and the return section 114 are the same, which improves the synchronization of the folding or expansion of the artificial plant 60. As shown in Fig. 28, when the two action points (X1, X2) have a large circumferential span, correspondingly the lengths of the forward section 112 and the return section 114 are different, and correspondingly the movement speeds of the forward section 112 and the return section 114are different, resulting in abnormal folding/expansion of the artificial implant.

In the existing technology, in order to improve the synchronization of folding and expansion of the artificial implant, a larger number of pulling wires (such as six or more pulling wires) are needed. However, it is necessary to consider whether the spatial arrangement of each pulling wire at the proximal end is allowed, as there may be friction or entanglement between the pulling wires that are too close. The threading method of this embodiment reduces the required number of pulling wires, thereby meeting the space requirements. The above control mechanism can be installed in the delivery system, which further includes a control handle located at its proximal end and an inner shaft assembly connected between the control handle and the control mechanism, wherein the inner shaft assembly includes the first shaft, the second shaft, and the third shaft described above.

In some embodiments, the delivery system further includes an outer sheath mounted around the inner shaft assembly. During the interventional delivery process, the distal end of the outer sheath wraps around the control mechanism and the artificial implant partially or entirely.

Referring to Figs. 11, 29, 30a and 30b, the present application also provides a locking mechanism for connecting artificial implant with delivery system, including a first shaft 21, a pulling wire 11, a third shaft 25, and a linkage assembly. The distal end of the first shaft 21 is fixed with a loading section 211, which is open towards the proximal end, configured for accommodating the distal portion of the artificial implant 60. The pulling wire 11 has a free end 111, which can pass through and wind around, or be detached from the artificial implant 60. The third shaft 25 is slidably mounted around the first shaft 21, and a locking assembly is provided on the third shaft 25. The artificial implant 60 is bound to the locking assembly by the pulling wire 11 during loading. The locking assembly includes a locking member 13 and a base 10, which have a locking state that cooperates with each other (corresponding to the first state of the pulling wire) and a unlocking state that disengages from each other (corresponding to the second state of the pulling wire), restricting the pulling wire 11 from detaching from the artificial implant 60 and allowing the pulling wire 11 to detach from the artificial implant 60, respectively. The relationship between the proximal end of the artificial implant and the pulling wire 11 and the locking assembly may refer to the previous embodiments. For example, the locking mechanism may further include a second shaft 23, which is slidably mounted between the first shaft 21 and the third shaft 25 and is controlled at the proximal end by the control handle. The proximal end of the pulling wire 11 is connected to the distal end of the second shaft 23.

The linkage assembly acts between the first shaft 21 and the locking member 13 to selectively link them together. It can be understood that, for the first shaft 21 and the locking member 13, there is an engaging state in which they cooperate with each other and the locking member 13 is driven to move by the first shaft 21, as well as a disengaging state in which they are separated from each other when the cooperation is released. The time for engagement should be after the accurate positioning and expansion of the artificial implant 60, and before the delivery system is ready for withdrawal. The way of engagement/disengagement of the first shaft 21 and the locking member 13 may be at least one of them moving relative to the other. Combining the above, for example, the locking member 13 is located at the distal end of the base, and the linkage assembly includes two mating portions, one mating portion is connected to the loading section 211 and the other mating portion is connected to the locking member 13. The axial movement of the loading section 211 can cause the two mating portions to engage or disengage. In one embodiment, after accurate positioning and expansion of the artificial implant 60, the loading section 211 located at the distal end is first moved towards the proximal end to engage with the locking member 13. At this time, the loading section 211 is inevitably located inside the artificial implant 60 in the axial position. Then, the first shaft 21 is rotated and the control of the pulling wire 11 is released through the linkage assembly. During the rotation operation, the proximal end of the loading section 211 is not easily or even interfered with the distal end of the artificial implant, for example, the loading section 211 is hung on the distal end of the artificial implant, and thus there is no need to separately set up a drive tube for the locking member, simplifying the structure.

The two mating portions are configured as a linkage key and a linkage groove that can slide relative to each other axially to separate. When the linkage key is inserted into the linkage groove, they are rotatably linked with each other. Of course, the two mating portions may be configured as a linkage key and a linkage groove that can rotate relative to each other to separate. When the linkage key is inserted into the linkage groove, they are linked with each other along the axial direction. Among them, there are multiple linkage keys, and the loading section and locking member are at least partially tubular, and the multiple linkage keys are arranged along the circumferential direction of the tubular part. Correspondingly, there are multiple linkage grooves.

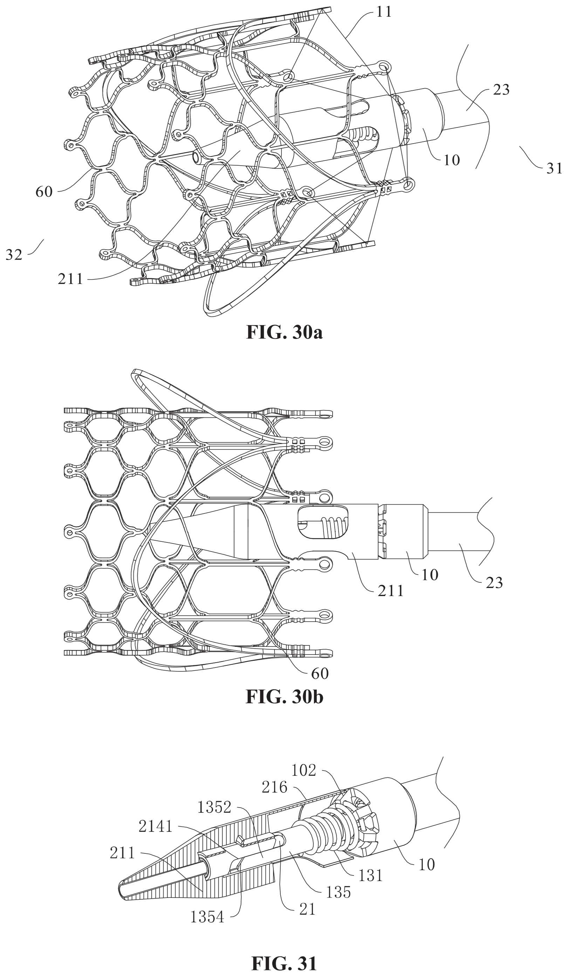

Referring to Figs. 31-34a, combined with the structure of the locking member 13 described above, in one embodiment, at least a part of the locking member 13 is configured as the connecting portion 135. The first shaft 21 and the connecting portion 135 are selectively matched through the linkage assembly to drive the locking member 13 to rotate relative to the base 10. The linkage key 1352 protrudes radially from one of the connecting portion 135 and the first shaft 21; and the linkage groove 2141 cooperates with the linkage key 1352 and is set on the other of the connecting portion 135 and the first shaft 21, and the linkage key 1352 and the linkage groove 2141 are formed at the end surface of the component where they are located. In the drawings, the linkage key 1352 is set at the distal end of the connecting portion 135, and the linkage groove 2141 is provided on the loading section 211. The rotational linkage between the linkage key 1352 and the linkage groove 2141 is manifested in that: in the circumferential direction, the side wall of the linkage groove 2141 is in contact with the side wall of the linkage key 1352 (as shown in Fig. 31). The connecting portion 135 is a cylindrical structure, and the linkage key 1352 is a sheet-like structure. In one embodiment, there are 2~4 linkage keys 1352 distributed along the circumferential direction, for example, there are two linkage keys 1352.

Preferably, the open side of the linkage groove 2141 is provided with an expansion structure 2142 for guiding the insertion of the linkage key 1352 into the linkage groove 2141, and the distal end surface of the linkage key 1352 is provided with a contraction structure 1354 for guiding the linkage key 1352 to insert into the linkage groove 2141.

The loading section 211 is nested with the connecting portion 135 in the engaged state, and the inner circumferential wall of the loading section 211 is provided with the linkage key 1352, so that the loading section 211 is inside the artificial implant in the engaged state. The inner circumference of the loading section 211 is provided with a support member 212, which is fixed to the distal end of the first shaft 21, and one of the matching portions (i.e., the linkage key) is provided on the support member 212. A limit structure is provided between the support member 212 and the loading section 211 to restrict their axial separation.

In one embodiment, the limit structure includes a positioning piece 2131 protruding radially from an outer circumference of the support member, and a positioning groove set on the inner circumferential wall of the loading section 211 to cooperate with the positioning piece 2131.

The support member 212 is a cylindrical structure, and the outer circumference of the support member 212 is partially flipped to form the positioning piece 2131. The support member 212 includes an outer cylinder 2130 connected to the loading section 211 and an inner cylinder 2140 configured with the linkage key and fixed to the first shaft 21. The inner cylinder and outer cylinders may be integrally formed as one piece or formed as separate structures and then connected together by means of, such as bonding. The outer circumference of the outer cylinder 2130 is partially flipped to form the positioning piece 2131, and the inner cylinder 2140 is concaved partially to form the linkage groove 2141.

The loading section 211 includes a guiding head 215 located at its distal end and connected to the first shaft 21, and a loading portion 216 connected to the guiding head 215 for accommodating the distal portion of the artificial implant. The support member 212 is set inside the guiding head 215. The distal end of the guiding head 215 gradually converges towards the distal end, and the proximal end surface of the guiding head 215 is a flat surface perpendicular to the axial direction. In the engaged state of the loading section 211, the proximal end surface of the loading portion 216 abuts the base 10. Specifically, in combination with the aforementioned embodiments, the proximal end surface of the loading portion 216 abuts the ribs 102 of the base 10. In one embodiment, the loading section 211 has a diameter equal to that of the base 10, and in the engaged state, the outer surfaces of the loading section 211 and the base 10 are flush.

Among them, the locking member will move towards the distal end during the rotation unlocking process. In one embodiment, when the loading section 211 and the locking member 10 are in the engaged state, there is an axial gap L1 between the linkage key and the linkage groove for the movement of the connecting portion towards the distal end. Similarly, the proximal end surface of the guiding head has the same axial gap L1 from the positioning portion 131. The axial gap L1 is greater than the movement distance of the locking member 13 towards the distal end.

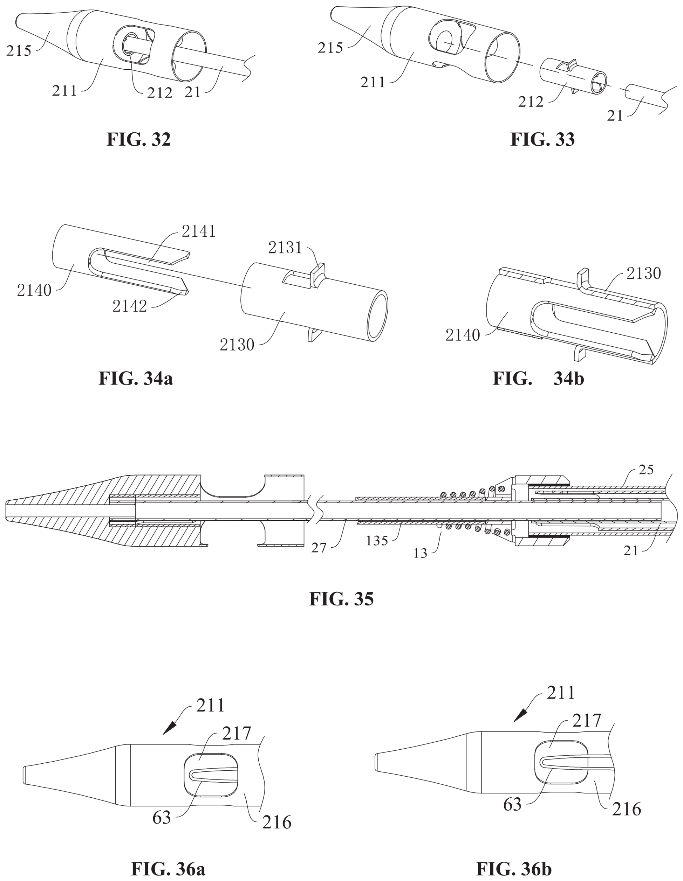

As shown in Fig. 35, the distal end of the first shaft 21 is fixed with a tubular-shaped extension tube 27 extending out of the base, and the locking member 13 is mounted around the outer circumference of the extension tube 27. The loading section 211 is set on the distal end of the extension tube 27. In one embodiment, the first shaft 21 is connected to and mounted around the proximal end of the extension tube 27, that is, the outer diameter of the first shaft 21 is larger than the outer diameter of the extension tube 27. The distal end of the first shaft 21 extends close to the base 10, and the proximal end of the first shaft 21 extends with constant diameter to connect the control handle. The larger diameter of the first shaft 21 facilitates force transmission and control. The smaller diameter of the extension tube 27 facilitates the arrangement of components such as the locking assembly at the distal end, the loading section, etc., reducing the overall size in the radial direction. The proximal end of the extension tube 27 extends into the base and is connected to the first shaft 21 fixedly. In the following embodiments, unless otherwise specified, the extension tube 27 is considered a part of the first shaft 21.

As shown in Figs. 35 to 38, an embodiment of the present application also provides a loading structure for an artificial implant. The artificial implant 60 includes an inner frame 61 configured with multiple arm portions 63 on its outer circumference. A first gap is formed between each arm portion 63 and the inner frame 61 to accommodate native tissue. The loading structure includes a first shaft 21 and a loading section 211, wherein the inner frame 61 in a compressed state is mounted around the outer circumference of the first shaft 21. The loading section 211 is tubular-shaped and fixed to the distal end of the first shaft 21. The loading section 211 opens towards the proximal end, and the entire inner frame 61or at least the distal portion of the inner frame 61 is accommodated in the loading section 211. The circumferential wall of the loading section 211 is provided with a pocket hole 217, and at least a part of the arm portion 63 is located at the pocket hole 217.

The loading section 211 binds the distal portion of the artificial implant 60, maintaining the artificial implant in a folding state to ensure safe intervention and delivery. At the same time, a part of the arm portion 63 enters the pocket hole 217, which is located in a portion of the loading section 211 configured with maximum radial space, i.e., the aforementioned loading portion 216. Compared to the existing structure that completely wraps artificial implants, the radial size of the loading section in this embodiment is smaller, which is conducive to interventional delivery.

In the loading state of the artificial implant, a radial relationship between the arm portion and the loading section, as well as the corresponding method of releasing the arm portion, are as follows: as shown in Fig. 36a, at least part of the arm portion 63 is located within the loading section 211. The loading section 211 moves axially to release its restraint. If there is a catheter sheath mounted around the loading section 211, the catheter sheath 70 needs to be moved first.

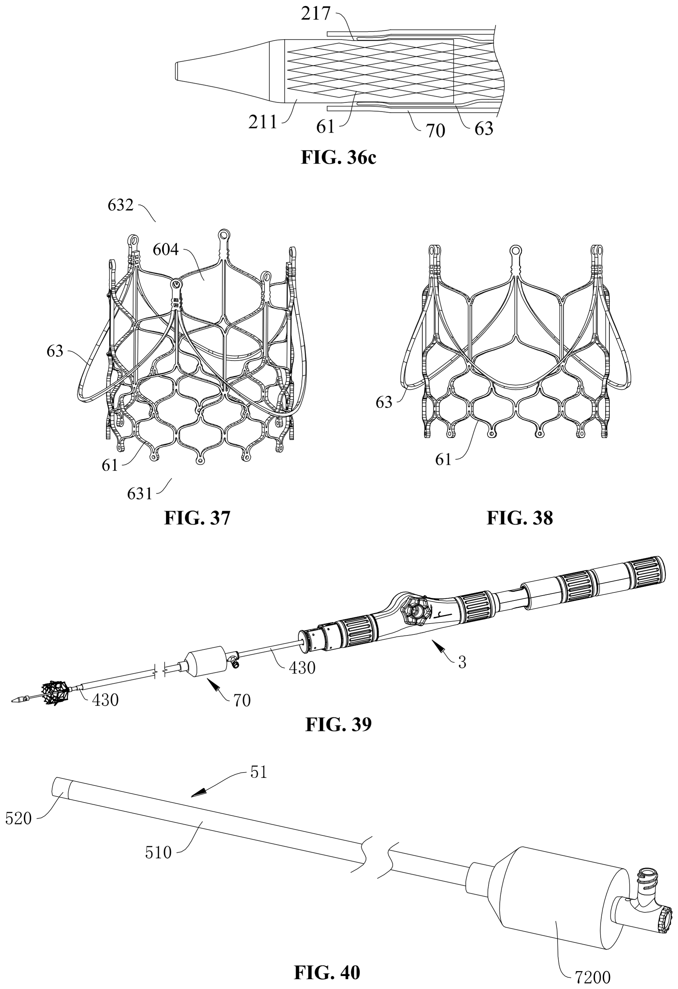

Alternatively, as shown in Figs. 36b and 36c, the loading structure includes a catheter sheath 70, which is slidably mounted on the outermost side of the inner shaft assembly, and the arm portion 63 is located between the loading section 211 and catheter sheath 70. After moving the catheter sheath 70, the restraint on the arm portion 63 can be released. In one embodiment, the distal portion of the artificial implant is located within the loading section, and the proximal portion of the artificial implant is located outside the loading section and within the catheter sheath.

The outflow side 632 of each arm portion 63 is fixedly connected to the inner frame 61, and the inflow side 631 expands to form a first gap with the inner frame 61, wherein the inflow side 631 is located at the pocket hole 217. The artificial implant includes an inner frame that defines a blood flow channel, and multiple arm portions are provided on the outer circumference of the inner frame. A first gap is formed between each arm portion and the inner frame to accommodate native tissue, and leaflets configured for controlling the blood flow channel are connected to the inner frame. The inner frame 61 and the arm portion 63 are formed separately and then fixed together or integrally formed as one piece. The inner frame 61 and the arm portion 63 are formed integrally by cutting a pipe blank. Along the axial direction of the pipe blank, the inflow sides of the inner frame 61 and the arm portion 63 are away from each other. Among them, the inner frame 61 is a radially deformable cylindrical structure with blood flow channel 604 defined therein. The leaflets are connected to the inner frame 61 to change the opening degree of the blood flow channel. The arm portion 63 is an integrated annular structure, and the outflow side 632 of each arm portion 63 is fixedly connected to the inner frame 61.

As shown in Figs. 39 to 40, an embodiment of the present application also provides an interventional delivery system for an artificial implant, including a catheter sheath 70 (i.e. the outer sheath mentioned above), a sheath adjusting assembly, an inner shaft assembly, and a control handle 3. The catheter sheath 70 is used to construct an interventional channel extending from the outside of the body to a position adjacent to the lesions. The proximal end of the catheter sheath 70 is provided with a fixed seat 7200 (located outside the body). The distal end of the sheath adjusting assembly can be controlled to change direction. The sheath adjusting assembly slides through the fixed seat 7200 and further extends towards the proximal end. The distal end of the inner shaft assembly is provided with a loading section for connecting the artificial implant 60, which is always exposed to the distal end of the sheath adjusting assembly. The control handle 3 is connected to the proximal ends of the sheath adjusting assembly and the inner shaft assembly. The control handle 3 is located at the proximal end side of fixed seat 7200 and the distance between it and the fixed seat 7200 is adjustable. The catheter sheath 70 of this embodiment is used to assist in the withdrawal of the artificial implant.

Specifically, the length of the catheter sheath allows it to reach a distant surgical site. During the intervention delivery process, the catheter sheath wraps around the control mechanism, proximal portion of the artificial implant, and/or the loading section as described in the above embodiments. The catheter sheath 70 includes a catheter body 51, which includes a main section 510 and a deformable section 520. The fixed seat 7200 is connected to the proximal end of the main section 510, and the deformable section 520 is located at the distal end of the main section 510. In one embodiment, the fixed seat 7200 is a hemostatic valve, which can adopt the existing structure.

The sheath adjusting assembly includes:
a bendable sheath 430, configured with a proximal end thereof being fixed to a control handle 3;
a sheath adjusting member, configured with one end thereof extending and fixed to the distal end of the bendable sheath 430 and acting on the catheter sheath, and the other end controlled by a sheath adjusting drive mechanism. The sheath adjusting member may be wires, pipes, or a combination of wires and pipes. For example, the sheath adjusting member is configured as adjusting wires fixed to the sheath tube, and the adjusting wires drive the bendable tube to bend the bendable sheath 430.

An embodiment of the present application also provides a control method for an interventional delivery system, which is used for interventional delivery of the artificial implant of the aforementioned embodiments. The delivery system further includes the control mechanism, the control handle, and the inner shaft assembly connecting the control handle, the control mechanism, and the loading section of the aforementioned embodiments. The control method includes:
expanding at least a part of the arm portion to make the first gap being aligned with native tissue;
driving the loading section 211 towards the distal end to disengage from the inner frame 61, making the distal portion of the inner frame 61 expand;
expanding the proximal portion of the inner frame 61 by releasing the pulling wire 11 exposed to the base, making the entire inner frame deform to an expected amplitude, and maintaining control of the inner frame 61 by the pulling wire 11 throughout the process;
moving the loading section 211 towards the proximal end to make the loading section 211 enter the blood flow channel 604; and
disconnecting the pulling wire 11 from the inner frame 61.

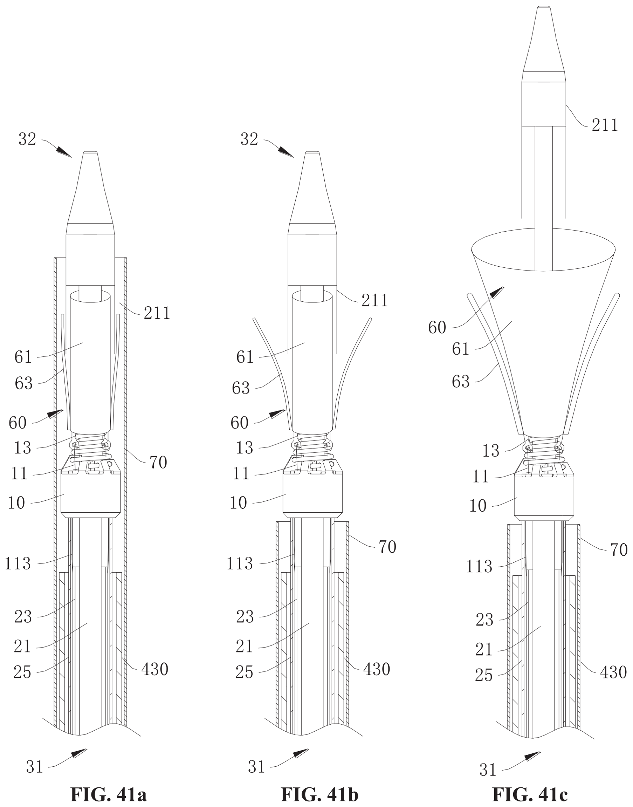

Specific details will be described in combination with the accompanying drawings: after the artificial implant is delivered to the predetermined position inside the body, the loading section 211 is driven towards the distal end, or the catheter sheath or its internal structure is driven, so as to make the loading section 211 and the catheter sheath move axially in opposite directions (depending on the different settings of the arm portion) until at least a part of the arm portion is released and expanded, the distal end of the inner frame is still bound by the loading section 211, and the first gap is aligned with the native tissue. This embodiment takes the scheme of setting the arm portion between the loading section and the catheter sheath 70 as an example and describes it with reference to the accompanying drawings:
As shown in Figs. 41a and 41b, by means of driving the catheter sheath 70 towards the proximal end or driving the internal structure of the catheter sheath towards the distal end, the constraint of the catheter sheath on the arm portion 63 is released, and thus the arm portions 63 expand radially and outwardly.

As shown in Fig. 41c, by means of pushing the first shaft 21 towards the distal end, the constraint of the loading section on the artificial implant is released, that is, the distal end of the inner frame is no longer constrained and expands radially and outwardly, and then it is prepared to expand the proximal end of the inner frame.

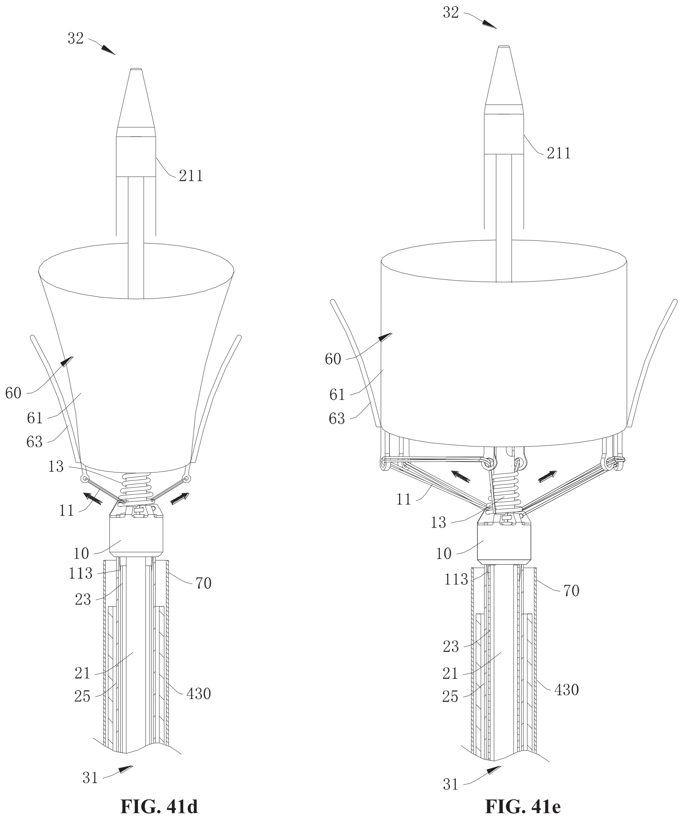

As shown in Fig. 41d and Fig. 41e, the third shaft 25 is kept stationary and the second shaft 23 is pushed towards the distal end, causing the control end 113 of the pulling wire 11 to move towards the distal end 32, gradually extending the exposed part of the pulling wire 11 out of the base, allowing the artificial implant 60 (mainly the proximal portion) to gradually expand. The pulling wire out of the base 10 will gradually extend in the direction of the arrow. The control end 113 continues to move until to the state shown in Fig. 41d, and the artificial implant 60 is in an expanding state (i.e., the expected amplitude). After confirming that it is compatible with the surrounding tissue as expected, the pulling wire can be released.

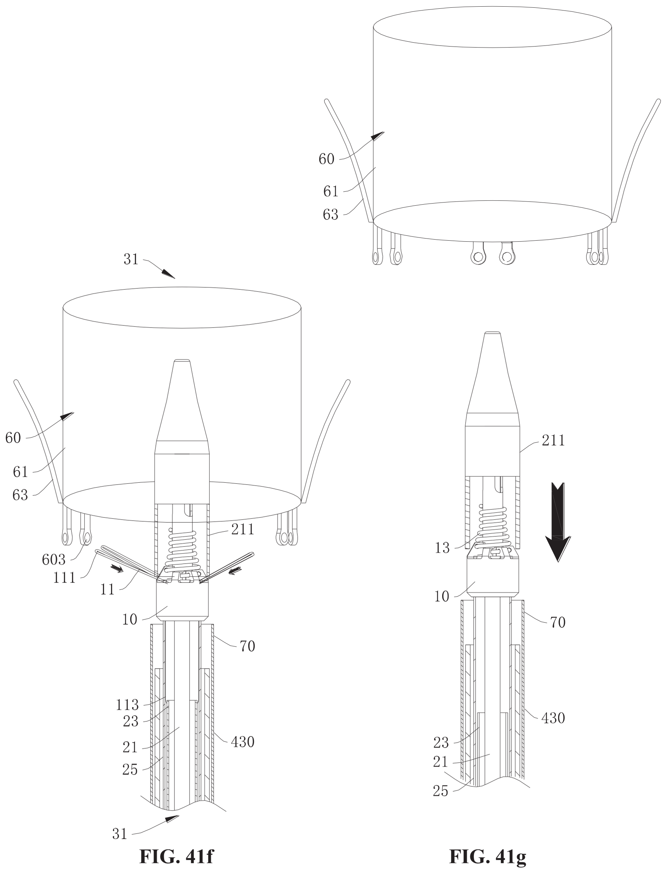

When releasing the pulling wire, as shown in Fig. 41f, the first shaft moves towards the proximal end until the loading section 211 enters the blood flow channel and is combined with the locking member 13 through the linkage assembly. The rotation of the first shaft 21 drives the locking member 13 to rotate relative to the base 10, and sequentially releases all of the pulling wires 11 until all free ends 111 completely detach from the locking member 13.

Then, the second shaft 23 is withdrawn towards the proximal end, causing the control end 113 of each pulling wire to move towards the proximal end 31, so that the free end disengages from the corresponding hole 603, i.e., the pulling wire completely disengages from the artificial implant, which can also be understood as releasing the artificial implant.

Finally, as shown in Fig. 41g, the entire delivery system is withdrawn to the outside of the body.

During the process of withdrawal the loading section 211 into the blood flow channel, the pulling wire remains in the first state to maintain the relative fixed spatial posture between the various components of the control mechanism and the artificial implant, guiding the loading section 211 to smoothly retract into the blood flow channel and reducing or eliminating the safety risk of the loading section 211 hanging on the distal end of the artificial implant during retraction.

In addition, before the pulling wire is unlocked, retraction may be done according to need. The retraction operation generally requires the combination of other components, such as the catheter sheath described in the previous embodiments. The specific operation includes: when the pulling wire is in the first state, the control end of the pulling wire is driven to move towards the proximal end, the proximal end of the artificial implant is retracted by the pulling wire, and then the remaining components inside the catheter sheath are moved towards the proximal end and retracted into the catheter sheath as needed, or the catheter sheath is pushed towards the distal end to receive the artificial implant.

In the initial stage of retraction, there is a certain gap between the distal end of the catheter sheath and the proximal end of the artificial implant, or the distal end of the catheter sheath and the proximal end of the artificial implant are already in a state of mutual contact. In this state, there is a significant force between the catheter sheath and the artificial implant, which is fed back to the control handle to make the surgeon feel a significant operational resistance. In this state, the artificial implant can be retracted by referring to the following embodiments of the telescopic assembly and push drive mechanism.

If there is a certain gap between the distal end of the catheter sheath and the proximal end of the artificial implant, a telescopic assembly can be used to directly drive the catheter sheath to move towards the distal end and quickly eliminate the gap. The following method can also be used to drive the inner shaft assembly to move towards the proximal end to eliminate the gap. Alternatively, the above two methods can be combined to eliminate the gap until the surgeon clearly feels the operational resistance, proving that the catheter sheath and artificial implant abut against each other.

It should be noted that the position of the axis in the drawings is not limited, and the control end can be very long or short, or the pulling wire can be directly connected to the control handle and directly retracted and extended. For the control of the pulling wire, the control end of the pulling wire may extend to and directly controlled by the control handle.

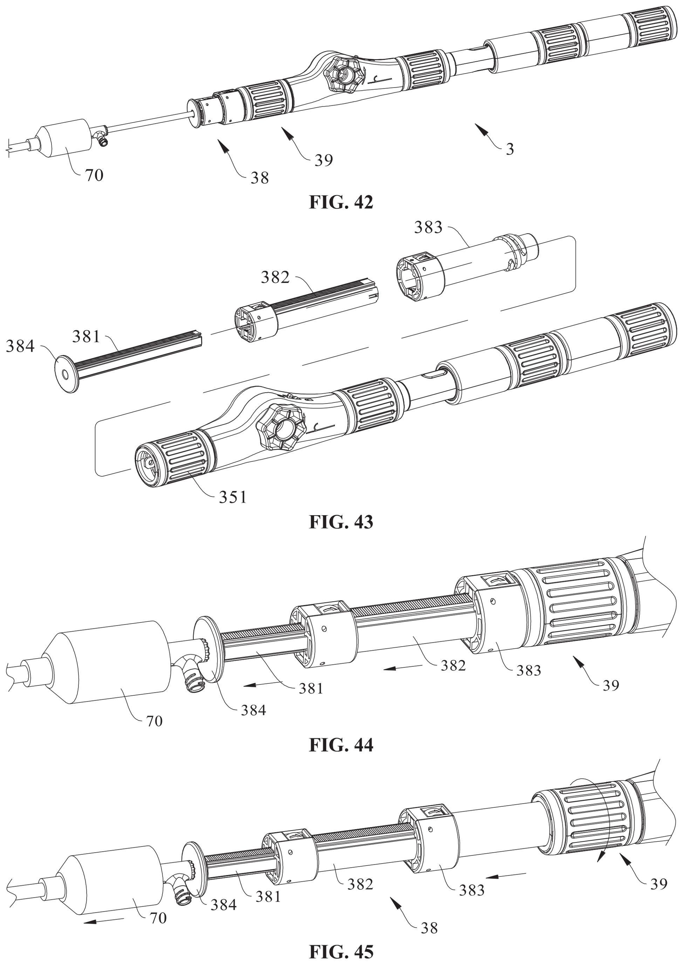

The above control handle can be a conventional control handle. This disclosure provides another type of control handle, as shown in Figs. 42-45. The control handle has opposite distal end and proximal end, as well as an axial direction extending between the proximal and distal ends. The distal end of control handle 3 is equipped with a push mechanism capable of telescoping in the axial direction. The push mechanism includes a telescopic assembly 38 and a push drive mechanism 39. The distal end of the telescopic assembly 38 has an abutment 384, and the proximal end of the telescopic assembly 38 is connected to control handle 3 in an axially movable manner. The push drive mechanism 39 is installed on control handle 3 and linked with the proximal end of telescopic assembly 38.

The telescopic assembly 38 is used to adjust the distance between the abutment 384 and the catheter sheath in the previous embodiments, and can keep the abutment in any position. The telescopic assembly 38 can maintain a distance between the abutment 384 and the catheter sheath, facilitating intervention in delivery operations. During withdrawal the artificial implant, the abutment 384 is driven to move towards the distal end until it abuts the proximal end of the catheter sheath, and then the inner shaft assembly is driven to move towards the proximal end relative to the catheter sheath 70 until the artificial implant is withdrawn into the catheter sheath 70.

Alternatively, the push mechanism 39 can act on the final level 383 to drive the telescopic assembly 38 to move further towards the distal end, and drive the catheter sheath to move relative to the inner shaft assembly towards the distal end until the artificial implant is withdrawn into the catheter sheath 70.

Alternatively, the withdrawal of the artificial implant can only be completed within a certain area of the body. Therefore, the telescopic assembly 38 is first extended to that area, and at this time, the distal end of the catheter sheath 70 is close to the control mechanism, reducing the distance between the catheter sheath and the artificial implant. Then, the inner shaft assembly is withdrawn until the artificial implant is also located in that area. Finally, the push drive mechanism 39 completes the withdrawal of the artificial implant according to the above operation.

Among them, the telescopic assembly 38 can move quickly to eliminate the distance between the abutment 384 and the catheter sheath, which belongs to coarse adjustment. The push drive mechanism 39 is controllable and precisely adjusts the axial movement distance of the telescopic assembly 38, which belongs to fine adjustment.

In one embodiment, the telescopic assembly 38 is configured in form of one level or multiple levels from the distal end to the proximal end. The distal end of the first level 381 is equipped with an abutment 384, and the proximal end of the final level 383 is linked with the push drive mechanism 39. The various levels of the telescopic assembly 38 are cylindrical structures and can be movably inserted in sequence to ensure distance adjustment while saving axial space and reducing the axial size of the control handle. For two adjacent levels, one is an outer cylinder and the other is an inner cylinder. When the telescopic assembly 38 extends, the inner cylinder moves towards the distal end relative to the outer cylinder. On the contrary, when the telescopic assembly 38 is shortened, the inner cylinder moves towards the proximal end relative to the outer cylinder. The telescopic assembly 38 has a first limit of maximum length and a second limit of minimum length. Among them, the telescopic assembly 38 includes a first level 381, an intermediate level 382, and a final level 383 from inside to outside.

In one embodiment, helical nti-retreat structures cooperating with each other set between adjacent levels. At least the movement of the inner cylinder relative to the outer cylinder towards the proximal end is restricted. During operation, the restriction of the anti-retreat structures needs to be released. For the movement of the inner cylinder relative to the outer cylinder towards the distal end, the anti-retreat structures can restrict it or only provide a certain resistance, for example, the restriction of the anti-retreat structures can be released under the action of an external force.

In one embodiment, a control method for the withdrawal of an artificial implant using an interventional delivery system is provided, including:
providing an artificial implant and an interventional delivery systems for delivering the artificial implant, the interventional delivery system including:
a catheter sheath, configured for constructing an interventional channel, a fixed seat being provided at the proximal end of the sheath;
an inner shaft assembly, configured with a loading section at its distal end, the artificial implant being at least partially located in the loading section and connected to the inner shaft assembly in a releasable manner before expansion;
a control handle, connected to the proximal end of the inner shaft assembly, the control handle being located at the proximal end side of the fixed seat, and the distal end of the control handle being provided with a push mechanism that can interact with the fixed seat;
at least a portion of the artificial implant maintains connection to the inner shaft assembly, and during withdrawal, the catheter sheath is driven to move relative to the inner shaft assembly, allowing the artificial implant to be received into the catheter sheath.

The proximal end and distal end of this embodiment are not strictly limited to installation positions, but can also be directional indicators, such as the push mechanism can extend to the control handle.

The relative motion between the catheter sheath and the inner shaft assembly can be either the catheter sheath moving towards the distal end, or the inner shaft assembly moving towards the proximal end, or the inner shaft assembly moving towards the proximal end and the catheter sheath moving towards the distal end, until the distal end of the catheter sheath approaches or contacts the artificial implant, eliminating the aforementioned gap, and waiting for the next operation. During withdrawal, the push mechanism or other components can be pressed against the proximal end of the fixed seat to provide sufficient force transmission, facilitating the withdrawal operation. The artificial implant, the catheter sheath, the inner shaft assembly, and the control handle are constructed according to the aforementioned embodiments. Specifically, in one embodiment, the distal end of the catheter sheath approaches or contacts the artificial implant, driving the push mechanism to abut against the fixed seat. First, the telescopic assembly is extended towards the distal end until the first level abutting the fixed seat. Then, the push drive mechanism is controlled to drive the entire telescopic assembly to move towards the distal end and act on the catheter sheath through the fixed seat to move it towards the distal end until the withdrawal of the artificial implant is completed.

The expansion of at least a portion of the artificial implant relative to the inner shaft assembly is understood as deformation to expand outwardly, or expansion after the distal end side detaching from the loading section or catheter sheath. In one embodiment, the artificial implant is connected to the inner shaft assembly through a pulling wire. During withdrawal, the pulling wire is kept in a tightened state to bring the proximal end side of the artificial implant together, which facilitates smooth entry of the proximal end of the artificial implant into the catheter sheath during withdrawal. Before withdrawal, if the artificial implant is in an expanding state, it is necessary to first tighten the pulling wire to bring the proximal end side of the artificial implant together. Among them, during the withdrawal process, the proximal end of the arm portion of the artificial implant enters the catheter sheath first, and the distal end of the arm portion enters the catheter sheath adaptively with the movement of the catheter sheath relative to the artificial implant.

In one embodiment, a loading method for carrying an artificial implant into a delivery system is provided, including:
providing an artificial implant and a delivery system, wherein the artificial implant include an inner frame configured with multiple arm portions on its outer circumference, and he delivery system includes:
a catheter sheath configured with a fixed seat at its proximal end;
an inner shaft assembly, extending movably through the catheter sheath, a loading section being provided at the distal end of the inner shaft assembly; and
a control the handle connected to the proximal end of the inner shaft assembly.

Specifically, the loading section is driven by operating the control handle, and the catheter sheath is operated to receive and wrap the artificial implant in an expanding state inside the loading section and the catheter sheath, so that the artificial implant is in a loading state.

Before loading, the artificial implant is in an expanding state and separated from the delivery system. In one embodiment, the pulling wire is used to pass through and wind around the proximal end of the inner frame to complete the connection between the artificial implant and the delivery system. Referring to the aforementioned structure, the delivery system further incldues a control mechanism, which includes a base connected to the inner shaft assembly, a locking member that movably cooperates with the base, and multiple pulling wires. The inner shaft assembly may refer to the previous embodiments.

One end of the pulling wire (i.e., the control end) is connected to and controlled by the control handle, and the other end of the pulling wire (i.e., the free end) extends out of the base and, under the operation of the control handle, elongates the length exposed out of the base to provide sufficient length for the of the pulling wire to pass through and wind around the proximal end of the inner frame and return to the corresponding locking area of the base, waiting to be locked to the base and locking member, wherein multiple of the pulling wires are locked one by one. During the locking process, the locking member rotates relative to the base.

Subsequently, the control handle is operated to drive the control end of the of the pulling wire to move towards the proximal end to tighten the of the pulling wire, i.e., reducing the length of the of the pulling wire exposed out of the base, thereby compressing the proximal end of the inner frame radially until it is close to the base. During the tightening process, all of the pulling wires are tightened synchronously.

Next, compression and loading operations are performed to the distal end of the inner frame:
In one embodiment, the distal end of the inner frame is compressed, and the control handle is operated to drive the loading section to move towards the proximal end to accommodate the distal end of the inner frame. At this time, the arm portions are still in an expanding state and not been accommodated by the loading section. Then, the control handle is operated again to drive the catheter sheath to move towards the distal end to receive the arm portions and the proximal end of the inner frame. Among them, the method of compressing the inner frame may be done by using a gripper in a cold water bath or manually compression.

In another embodiment, the inner frame and the distal ends of the arm portions are compressed, and the control handle is operated to drive the loading section to move towards the proximal end to receive the distal end of the inner frame and at least the distal ends of the arm portions. Then, the control handle is operated again to drive the catheter sheath to move towards the distal end for receiving the proximal end of the inner frame and the exposed parts of the arm portions. Among them, the method of compressing the inner frame and the arm portion may be done by using a pressure gripper in a cold water bath or manual compression. In one embodiment, the arm portions avoid the loading section or are placed on the outer circumference of the loading section, and the distal end of the catheter sheath abuts the loading section in the axial direction, or wraps around the proximal end of the loading section. In one embodiment, the method of moving the catheter sheath towards the distal end includes driving the catheter sheath to push the fixed seat towards the distal end relative to the control handle.

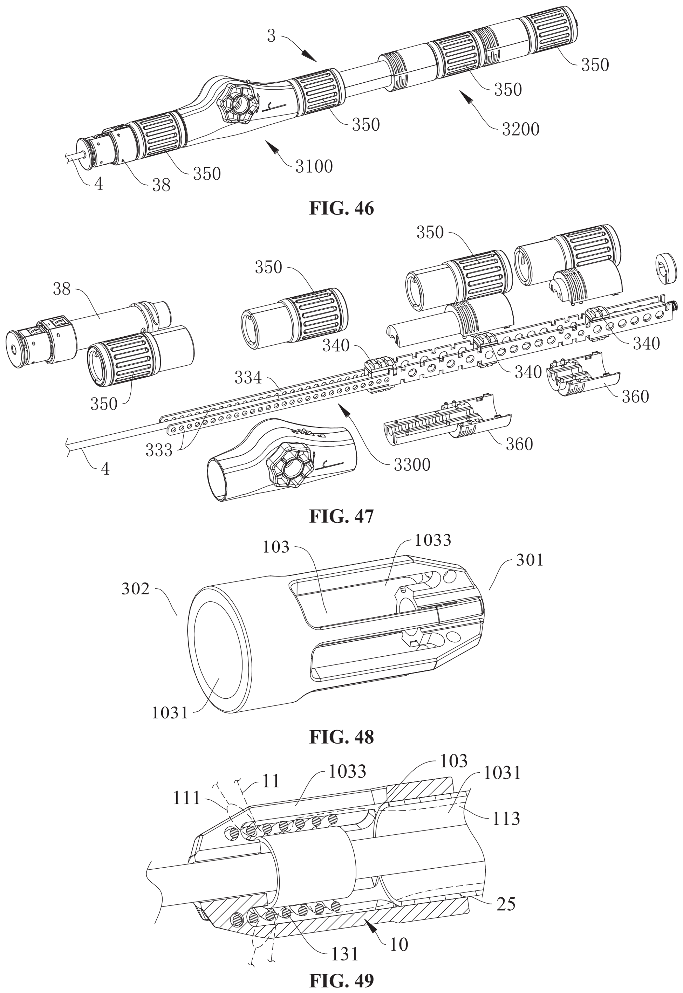

In the above two embodiments, the loading section has a pocket hole, and during the process of receiving the distal end of the inner frame, it also includes first aligning the pocket hole with the circumferential direction of the arm portion. The method of aligning in circumferential direction includes operating the control handle to drive the loading section to rotate in the circumferential direction. As shown in Figs. 46 to 63, the present application also provides a control handle 3, which is connected to a control catheter assembly 4 and includes:
a support body 3300, including two rigid bars 333 arranged side by side, with a guide channel 334 defined between the two rigid bars 333;
a plurality of drive mechanisms, at least two of which include a transmission member 340 that is slidably mounted in the guide channel 334, and a driving sleeve 350 that is rotatably mounted around the outer circumference of the support body 3300 and threaded with the transmission member 340.

The support body is configured as two rigid bars, on the one hand, the radially opposite sides of the guide channel are penetrated, which facilitates the installation of the transmission member along the radial direction; at the same time, the guide channel is also penetrated in the axial direction, which meets the motion requirements of the transmission member. Moreover, compared with the existing support structure, the strip-shaped structure is more convenient for processing and is not limited by materials. For example, the rigid bars 333 are metal bars, which effectively improve the structural strength of the support body.

In addition, if the number of components in the control mechanism increases or the operation becomes more complex, such as an increase in the number of transmission members and driving sleeves, rigid bars can be extended along the axial direction to meet the installation needs of excess transmission members and driving sleeves. The installation methods between each transmission member and the support body, and between each driving sleeve and the support body, are the same, achieving modular installation without sacrificing its own structural strength to meet installation requirements.

In one embodiment, the length of the two rigid bars 333 accounts for at least 75% of the total length of the control handle 3, where the length of the two rigid bars 333 is the length between two axial ends, and the total length of the control handle is the length of the control handle in a fully unfolding state, i.e., starts from the distal end surface of a first driving sleeve 351 and ends at the proximal end surface of a force applying component 355 described in the following embodiment.

In one embodiment, the rigid bar 333 is provided with multiple weight reduction holes to reduce the weight of the support body and decrease the contact area between the support body and the transmission member, thereby reducing friction and making the movement of the transmission member smoother.

In one embodiment, the distal end of the rigid strip 333 extends into the telescopic assembly 38, for example, the distal end of the rigid strip 333 extends close to the first level.

In another embodiment, the catheter assembly 4 includes a first group and a second group that are arranged in a movable manner from the outside to the inside. The control handle 3 includes a first handle 3100 and a second handle 3200. The first handle 3100 is used to connect the first group, and the second handle 3200 is used to connect the second group. Both the first handle 3100 and the second handle 3200 include a support body 3300, and each support body 3300 includes two rigid bars 333 arranged side by side. The first handle 3100 and the second handle 3200 can share the same support body (as shown in Fig. 46), or the support bodies of the first handle 3100 and the second handle 3200 are not shared. The first handle 3100 and the second handle 3200 are far away from each other, and the longest distance between the first handle 3100 and the second handle 3200 corresponds to the fully unfolding state of the control handle.

In one embodiment, the control handle 3 includes a mounting seat 360 fixedly connected to the two rigid bars 333, and the driving sleeve 350 is embedded between the rigid bars 333 and rotatably cooperates with the mounting seat 360. The mounting seat 360 provides the installation foundation for the driving sleeve while maintaining relative fixation between the two rigid bars. The present application also provides a delivery system, including a catheter assembly loaded with an artificial implant at the distal end, a control handle connected to the proximal end of the catheter assembly to control the catheter assembly, and the catheter sheath 70 of the above embodiments. The catheter assembly can be inserted into the catheter sheath through a hemostatic valve. For the specific structure of the catheter assembly and the control handle, existing technology can be used. Moreover, due to the fact that the deformation section can assist in the withdrawal of the artificial implant after switching to the expanding state, the structure of the catheter assembly can be further simplified. For example, compared to conventional catheter assembly, the outermost pipe fittings can be omitted.

The catheter sheath in this application is long to reach a relatively distant surgical site, such as extending to the ascending aorta, thereby constructing a longer channel for the delivery of the catheter assembly and the artificial implant.

In the following description, it mainly provides other embodiments for the structure of the locking member and the base. For the working principle, it can be combined with the previous embodiments. The proximal handle control can be combined with conventional technology or the previous embodiments.

In one embodiment, another configuration of locking member is provided. As shown in Fig. 48, the base 10 has opposite distal end 301 and proximal end 302, as well as an axial direction extending between the distal end 301 and proximal end 302. The interior of the base 10 has a first cavity 103, and the proximal end side of the base 10 has a first opening 1031 communicated with the first cavity 103, and the outer circumferential surface of the base 10 has a second opening 1033 communicated with the first cavity 103.

As shown in Fig. 49, the pulling wire 11 extends through the first cavity 103. One end of the pulling wire 11 (i.e., the control end 113) extends through the first opening 1031 towards the proximal end to extend out of the base 10. The other end of the pulling wire 11, i.e., the free end 111, extends out of the base 10 through the second opening 1033 to connect the artificial implant.

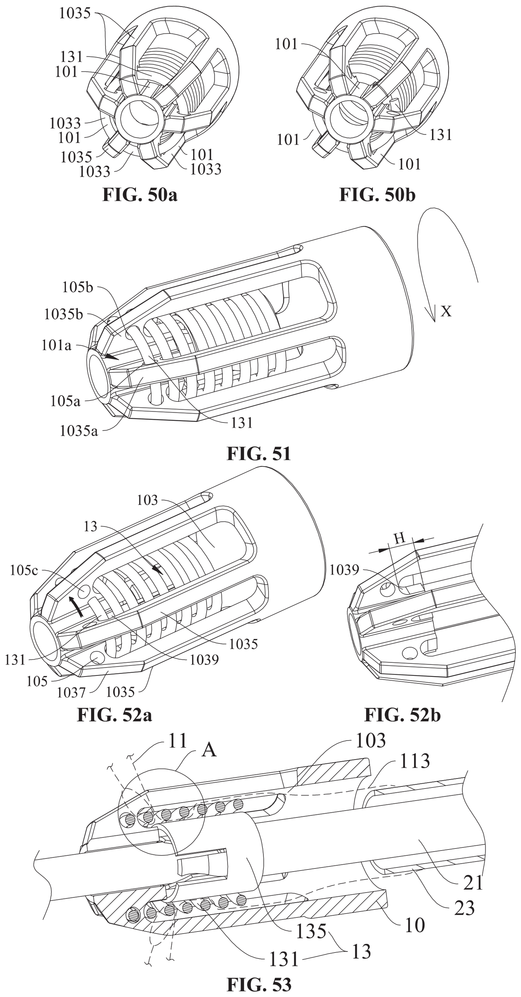

In this embodiment, referring to Figs. 50a and 50b, the locking area 101 is located at the second opening. Along the circumferential direction of the base 10, there are multiple locking areas 101 arranged at intervals, and the positioning portions 131 engages into or disengages from the locking areas 101 in sequence with the movement of the locking member 13.

In one embodiment, as shown in Fig. 49, the control mechanism further includes a third shaft 25 fixedly connected to the base 10. The proximal end of the third shaft 25 extends to and is controlled by the control handle. The third shaft 25 can keep the base 10 relatively fixed in the circumferential direction, so that the positioning portion 131 can rotate relative to the base 10.

In one embodiment, the base 10 is provided with multiple separating members arranged along the circumferential direction, and adjacent separating members define the second opening 1033. Therefore, there are multiple second openings 1033 arranged at intervals along the circumferential direction of the base 10, and the separating members are configured as ribs 1035 as shown in Figs. 50a and 50b. When the positioning portion 131 moves in the first direction (as shown by X in Fig. 51), the free end 111 switches from the second state to the first state, and the locking hole 105 penetrates the rib 1035 along the first direction. The locking hole 105 has opposite forward and backward openings, wherein the forward opening faces the first direction.

In the preferred embodiment, all of the ribs 1035 (such as ribs 1035a and 1035b, etc.) are provided with locking holes 105, configured for rotataly extending through of the positioning portions 13 in sequence and provideing certain motion guidance. During the process of rotataly inserting the locking member into each locking hole, the head end 1313 finally abuts against the side wall 1034 of one of the ribs 1035, or is inserted into the corresponding locking hole, playing a limiting role.

When the positioning portion 131 is in a helical shape with multiple turns, the opening position of the locking hole 105 of each rib 1035 is adapted to the positioning portion 131. Among them, there are one or multiple locking holes 105 on the same rib 1035. The multiple locking holes 105 are arranged in sequence along the extension direction of the rib 1035 where they are located.

A side of the rib 1035 facing the first cavity 103 is the inner side, and each rib 1035 is provided with a guide groove 1039 on the inner side for extending the positioning portion 131. The axial penetration of the guide groove 1039 allows the positioning portion 131 to rotate through, and the proximal end side of the guide groove 1039 is open for the positioning portion 131 to be inserted into and position. After the locking member 13 rotates, the positioning portion 131 is inserted into the first locking hole 105c (as shown in Fig. 52a). In one embodiment, the groove depths (H in Fig. 52b) of the guide grooves corresponding to the ribs are different, satisfying the rotation guidance of the helical positioning portion 131.

Referring to Fig. 52a, in one embodiment, one section of the rib 1035 along the axial direction is a bending section 1037 extending radially and inwardly, and the locking hole 105 is located on the bending section 1037 of the corresponding rib 1035. The bending section 1037 is located at the distal end, as a whole forming a chamfered or rounded structure on the distal side of the base, which serves as a guide for the folding of the artificial implant.

Based on the rotation characteristics of the locking member and the circumferential distribution of the pulling wires, the positioning portion 131 is rod-shaped, which can be a straight rod or curved rod, with a quantity of one and matched with the free ends 111 of all pulling wires 11. This eliminates the axial dimension change of the control mechanism while retains the function of original locking members to be matched with the pulling wires one by one.

In one embodiment, the base 10 is provided with a locking hole 105, and an open side of the locking hole 105 faces the circumferential direction of the base 10. The positioning portion 131 has a curved shape and extends around the axis of the base. The positioning portion 131 is inserted or removed from the locking holes 105 with the rotation of the locking member 13. Curved shape can fully utilize the circumferential space, maintain the necessary length of the locking member, and achieve control of the free ends of all pulling wires by the same locking member.

As shown in Fig. 53, at least a part of the locking member 13 is configured as the connecting portion 135 located in the first cavity 103. The control mechanism further includes a first shaft 21 that is in transmission cooperation with the connecting portion 135 to drive the locking member 13 to rotate relative to the base 10. The proximal end of the first shaft 21 is connected to and controlled by a control handle.

In one embodiment, the pulling wire 11 has a control end 113 opposite to the free end 111, wherein the control end 113 is movable relative to the base 10. The control mechanism further includes a second shaft 23 connected to the control end 113, wherein the second shaft 23 is tubular and movable mounted around the first shaft 21. The second shaft 23 moves to drive the pulling wire 11 to gradually retract or expand the artificial implant.

In some embodiments, the positioning portion 131 at least includes an arc-shaped structure that cooperates with the base. For example, as shown in Figs. 53-55, the positioning portion 131 is a helical structure that winds at least once. For example, the helical structure is a multi-turn structure, and the multiple turns are arranged along the axial direction (circumferential direction of the base). The generatrix of the helical structure can be a diagonal line (away from or near the axis of the axis) or a straight line. For example, the generatrix of the helical structure is a straight line, which is able to avoid increasing the radial size or reducing the fitting space between the connecting portion 135 and the first shaft 21.

In this embodiment, the head end 1313 of the positioning portion 131 is matched with the base 10 and the pulling wires, and a position where the tail end 1311 of the positioning portion 131 is located is fixed to the connecting portion 135, for example, fixed to the outer circumference or distal end of the connecting portion 135. The connection method may be direct welding of the ends or welding fixation after partial overlapping.

The connecting portion 135 is tubular, and its interior is used to accommodate other components, such as the first shaft 21. In order to improve the connection strength and avoid the extension of components, the positioning portion 131 can be helical wound around the outer circumference of the connecting portion 135. The connecting portion 135 and a section of the positioning portion 131 adjacent to the tail end 1311 are located in the first cavity 103 and are welded and fixed to each other.

The connecting portion 135 is fixed at the distal end of the first shaft 21, or can be axially separated from the distal end of the first shaft 21.

As shown in Figs. 53 to 55, in one embodiment, the positioning portion 131 is a multi-turn helical structure, with axial gaps defined between adjacent turns. An internal space 1317 is enclosed by the helical structure, and at least one section of the pulling wire **11** is a circuitous section 117. In the first state, the circuitous section 117 is located in the internal space 1317, and two ends of the circuitous section 117 extend outside the internal space 1317 through corresponding axial gaps. The circuitous section 117 forms a force point (first force point) with the positioning portion 131, and the free end 111 is mounted around a turn of the positioning portion 131 adjacent to the first axial gap 1315b and serves as the second force point, so that the second shaft 23 acts on the artificial implant for folding.

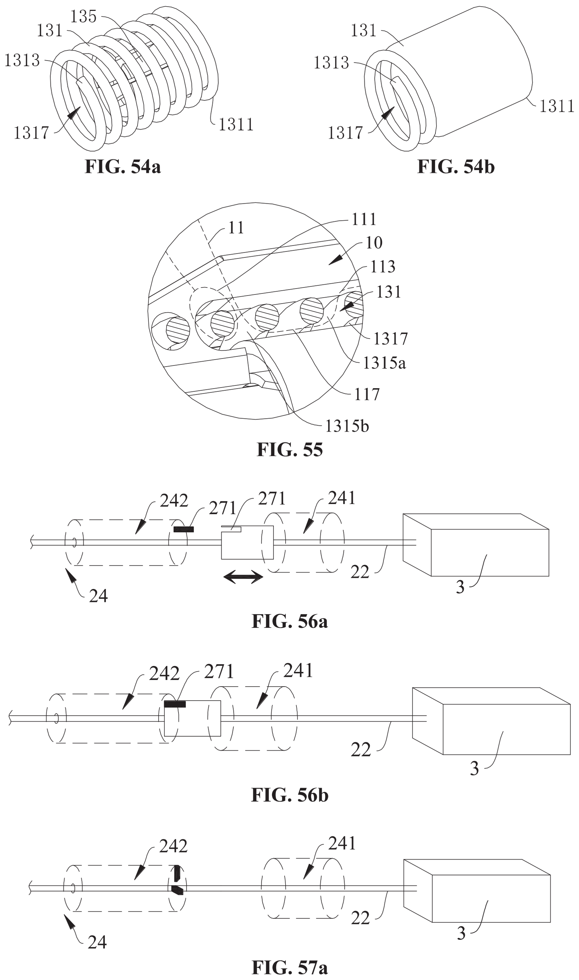

The number of turns of the positioning portion 131 is at least one and a half turns or two turns or more, for example, as shown in Fig. 54a, there are more than two turns; or, as shown in Fig. 54b, there are one and a half turns.

In the first state, the pulling wire 11 extends from the control end 113 to the outer side of the positioning portion 131, through the second axial gap 1315a and bends to the inner side of the positioning portion 131, and then extends from the second axial gap 1315a to the first axial gap 1315b at the distal end, through the first axial gap from the inside to the outer side and bends to the outside of the positioning portion 131. After winding around the artificial implant, the free end 111 is mounted around the positioning portion 131. The circuitous section 117 is located between the first axial gap 1315b and the second axial gap 1315a. A portion of the pulling wire 11 extending from the control end 113 to the second axial gap 1315a is located between the positioning portion 131 and the base 10, as far away as possible from the first shaft 21, reducing the impact on the cooperation between the first shaft 21 and the connecting portion 135, and facilitating the fixed connection between the connecting portion 135 and the positioning portion 131.

In another embodiment, the present application also provides a control mechanism having a distal end and a proximal end that are opposite to each other, and an axial direction extending between the proximal end and distal end, the control mechanism including:
an intervention component 24, including a first intervention component 241 and a second intervention component 242;
a transmission member 22 configured with a distal end thereof being fixedly connected to the first intervention component 241;
a control handle 3, connected to and controlling the proximal end of the transmission member 22; and
a linkage structure including two mating portions 271 being selectively linked, one of which is fixed to the second intervention component 242 and the other is fixed to the transmission member 22. During the movement of the transmission member 22, the linkage state of the two mating portions is switched.

When the two mating portions are in a linked state, the second intervention component 242 is driven to rotate or move linearly. For example, when the transmission member 22 slides in the axial direction, the two mating portions are linked or unlinked. As shown in Fig. 56a, the two mating portions 271 are in the unlinked state, and the transmission member 22 slides axially to switch the two mating portions 271 to the linked state, as shown in Fig. 56b. The transmission member 22 can drive the second intervention component 242 to rotate.

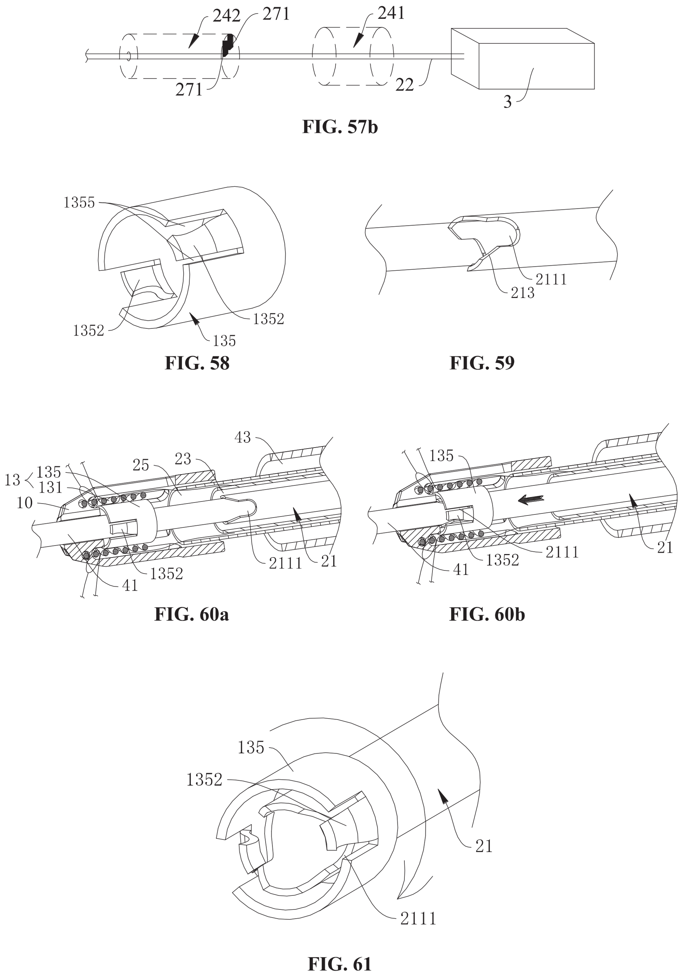

Alternatively, when the transmission member 22 rotates, the two mating portions are linked or unlinked. As shown in Fig. 57a, the two mating portions 271 are in the unlinked state, and the transmission member 22 rotates to switch the two mating portions 271 to the linked state, as shown in Fig. 57b. The transmission member 22 can drive the second intervention component 242 to slide.

The two mating portions are configured as a linkage key and a linkage groove that can slide axially to separate. When the linkage key is inserted into the linkage groove, they are rotatably linked with each other. The two mating portions may also be configured as a linkage key and a linkage groove that can rotate relative to each other to separate. When the linkage key is inserted into the linkage groove, they are linked with each other in the axial direction. Among them, there are multiple linkage keys, and a part of the intervention component is tubular. The multiple linkage keys are arranged along the circumferential direction of the tubular part. Correspondingly, there are multiple linkage grooves.

In the drawings, along the axial direction, the first intervention component 241 is located at one side (proximal end side) of the second intervention component 242. In an initial state or a linked state, the first intervention component 241 and the second intervention component 242 can also be partially nested with each other.

Based on common application scenarios, the first intervention component and the second intervention component each include the following independent components:
an inner shaft assembly 41, configured for carrying the artificial implant (such as artificial heart valve) before release;
an outer sheath which can wrap around the artificial heart valve before release;
a base connected to the artificial heart valve before release and axially limited to each other;
a locking member configured for limit the artificial heart valve before release inside the base;
a balloon body configured for carrying the artificial heart valve, and capable of expanding the artificial heart valve; and
a pulling wire connected to the artificial heart valve, configured for controlling the release process of the artificial heart valve.

Referring to Figs. 58-60b, combined with the structure of the locking member 13 mentioned above, in one embodiment, the locking member 13 serves as the second intervention component, and the first shaft 21 serves as the first intervention component. At least a part of the locking member 13 is configured as the connecting portion 135, and the first shaft 21 is in transmission cooperation with the connecting portion 135 to drive the locking member 13 to rotate relative to the base 10. The connecting portion 135 is tubular, and a pipe wall of the connecting portion 135 is configured with a rotary linkage structure that cooperates with the distal end of the first shaft. When the first shaft 21 moves axially relative to the connecting portion 135, it engages with or disengages from the rotary linkage structure. Among them, the connecting portion 135 is located inside the base 10, which is advantageous for coupling with the first shaft 21 in radial space.

The rotary linkage structure includes two mating portions for selective linkage, namely:
a linkage key 1352, protruding radially from one of the connecting portion 135 and the first shaft 21;
a linkage groove 2111, in cooperation with the linkage key 1352, being set on the other of the connecting portion 135 and the first shaft 21, and is open at the end surface of the mating portion where it is located. For example, in Figs. 60a and 60b, the linkage key 1352 is located at the connecting portion 135 (for example, at the proximal end or distal end of the connecting portion 135), and the linkage groove 2111 is located at the first shaft 21 with an open side facing the distal end. The linkage key 1352 or the linkage groove 2111 slides along the axial direction to switch the linkage state. The rotational linkage between the linkage key 1352 or the linkage groove 2111 is manifested in the circumferential direction, where the side wall of the linkage groove 2111 is in contact with the side wall of the linkage key 1352 (as shown in Fig. 61).

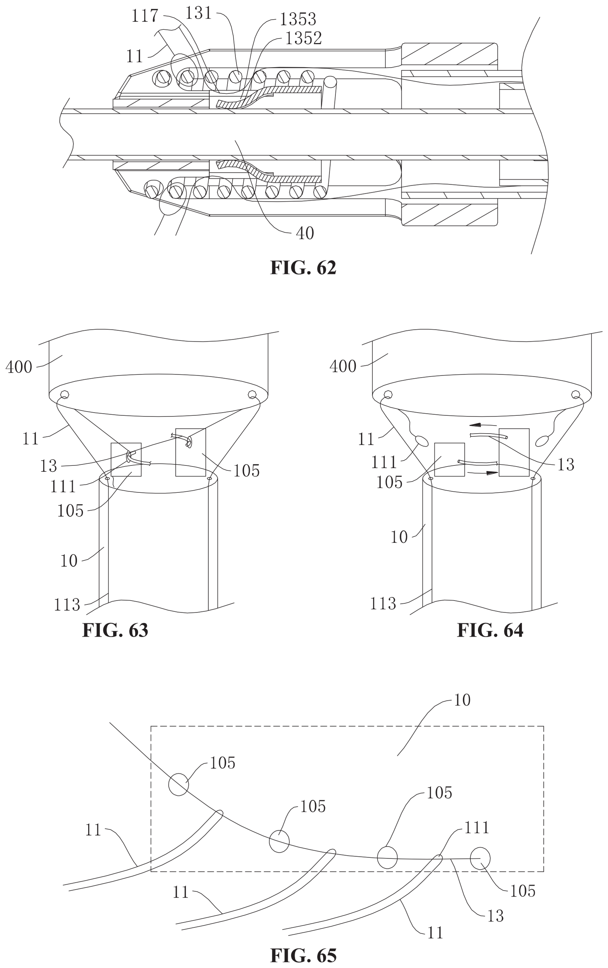

Preferably, as shown in Fig. 62, open side of the linkage groove 2111 is provided with an expansion structure 213 for guiding the insertion of the linkage key 1352 into the linkage groove 2111. The linkage groove 2111 is set at the distal portion of the first shaft 21, as shown in Figs. 60a, 60b, and 62, a pipe 40 extending out of the base is fixed at the distal end of the first shaft 21, and the linkage key 1352 is mounted around and abuts against the outer circumference of the pipe 40, maintaining the radial positioning between the locking member 13 and the base 10, and facilitating insertion into the linkage groove 2111.

In one embodiment, as shown in Fig. 62, the pipe wall of the connecting portion 135 deforms partly to form the linkage key 1352. An avoidance zone 1353 is formed on the outside of the linkage key 1352. The avoidance zone 1353 exists between the linkage key 1352 and the positioning portion 131, and the circuitous section 117 of the pulling wire 11 can be placed within the avoidance zone 1353 to facilitate the extending of the pulling wire 11.

The specific formation method of the linkage key 1352 includes: as shown in Fig. 58, the pipe wall of the connecting portion 135 is provided with paired cutouts 1355 near the proximal end surface, and the part between the paired cutouts 1355 is folded radially and inwardly to form the linkage key 1352. The connecting portion 135 is a cylindrical structure, and the linkage key 1352 is a sheet-like structure. In one embodiment, there are 2-4 linkage keys 1352 distributed along the circumferential direction. For example, as shown in Fig. 58, there are two linkage keys 1352. In another embodiment, another structural of the locking member is provided, and other embodiments in the disclosure can be used for the handle. As shown in Figs. 63-67, the present application provides a control mechanism for an artificial implant, including a base 10, a pulling wire 11, and a locking member 13. The base 10 has a distal end 32 and a proximal end 31 opposite to each other, as well as an axial direction extending between the distal end 32 and proximal end 31. The proximal end, distal end, and axial direction are also applipulling wire to other components of the control mechanism, as well as the control handle and the delivery system of the following embodiments, unless otherwise specified.

The base 10 is provided with multiple locking holes 105 arranged in a staggered manner along the axial direction. The pulling wire 11 has a free end 111 that can pass through and wind around, or be detached from the artificial implant 400. The free end 111 is the end where the pulling wire 11 first passes through and finally detaches from the artificial implant 400, and can also be understood as the farthest end when the pulling wire 11 is straightened. The pulling wire 11 also has a control end 113 opposite to the free end 111, which can also be understood as the closest end of the pulling wire 11 when it is straightened. The control end 113 can be directly fixed to the base 10 or extend towards the proximal end in a controllable manner. There is an area between adjacent locking holes 105 where the free end 111 of the pulling wire 11 is combined with the locking member 13. The locking member 13 is rotatably engaged with the base 10, and during the rotation process, the locking member 13 enters or exits the locking holes 105 to lock or unlock the free end 111 of the pulling wire 11 (hereinafter referred to as release).

Specifically, the free end 111 of the pulling wire 11 has a first state (as shown in Fig. 63) in which it is locked and restricted to the base 10 by the locking member 13, and a second state (as shown in Fig. 64) in which it is released and unrestricted by the locking member 13. The locking member 13 cooperates with the free end 111 of the pulling wire 11 to switch the state of the free end 111. In the first state, the free end 111 is combined with the locking member 13 and the locking member 13 is matched with the base 10 to restrict the pulling wire 11 from disengaging from the locking member 13. At this time, the free end 111 can be understood as being relatively fixed to the locking member 13 or the base 10. In this state, the pulling wire 11 is always connected to the artificial implant 400, and the expansion process (i.e. expansion degree) of the artificial implant is adjusted based on the length of the pulling wire 11 exposed out of the control mechanism, as well as the expansion/folding speed of the artificial implant is controlled based on the rate of change of the pulling wire. When necessary, the pulling wire 11 can also be used for the withdrawal of artificial implants. For example, when the pulling wire 11 is in the first state, the control end 113 can be operated to reduce the length exposed out of the control mechanism.

In the second state, the free end 111 is detached from the base 10. At this time, the free end 111 can be understood as being released. After the pulling wire 11 is released and detached from the artificial implant, the control end 113 is driven to move the pulling wire 11 towards the proximal end to withdraw the pulling wire 11, such as reducing the length of the pulling wire 11 exposed to the outer circumference of the base, preferably the pulling wire 11 is entirely accommodated inside the base. After the release of the pulling wire 11, the mutual connection between the artificial implant and the control mechanism is correspondingly released, and then the control mechanism as a whole can be withdrawn to the outside of the body, leaving the artificial implant in the predetermined position inside the body.

When the locking member 13, the base 10, and the pulling wire 11 are pre-assembled out of the body, the extension path of the pulling wire 11 enters the proximal end of the base, and then extends out from the distal end or the side wall of the base and extends through and wraps around the artificial implant before being combined with the locking member 13. For the combination of the pulling wire 11 and locking member 13, the free end 111 of the pulling wire 11 is first combined with the locking member 13 in the corresponding area of base 10 and thus in the first state. After that, locking member 13 continues to rotate to engage into the next locking hole 105, closing the channel for the pulling wire 11 to detach from locking member 13.

Based on Figs. 63 to 65, the multiple locking holes 105 of the base 10 in this embodiment are arranged along the axial direction, that is, the combination positions of the free ends 111 of the pulling wires 11 and the base 10 offset from each other in the axial direction, thereby reducing spatial interference between the pulling wires 11 and facilitating assembly of the pulling wires, the base, and locking member, as well as movement between the pulling wires.

Among them, the artificial implant 400 is generally cylindrical-shaped, the multiple locking holes 105 are arranged in sequence along the circumferential direction of the base, so that the pulling wires 11 are arranged along the circumferential direction and connected to various positions in the circumference of the artificial implant 400, maintaining uniform stress distribution in the circumference of the artificial implant 400 and keeping the length of the pulling wires 11 basically consistent, which is conducive to unified control of the length of all pulling wires 11 exposed out of the base, and expand the spatial distance between the pulling wires 11, further facilitating assembly and reducing interference between each other. In one embodiment, the axial positions of the multiple locking holes 105 are different. Specifically, the multiple locking holes 105 are arranged along a helical path that extends around the axis of the base. During the rotation of the locking member 13 around the axis of the base, its rotation path is constrained by the locking holes 105, and it sequentially enters each locking hole 105 and undergoes displacement along the axis of the base. This axial displacement can reduce or eliminate the axial protrusion of the locking member 13 relative to the base 10. The helical arrangement allows the locking holes 105 at different positions in the circumferential direction to be displaced in the axial direction, reducing spatial interference between the pulling wires 11, optimizing the overall meridian size, and further highlighting the synergy between the motion characteristics of the base structure and the locking member.

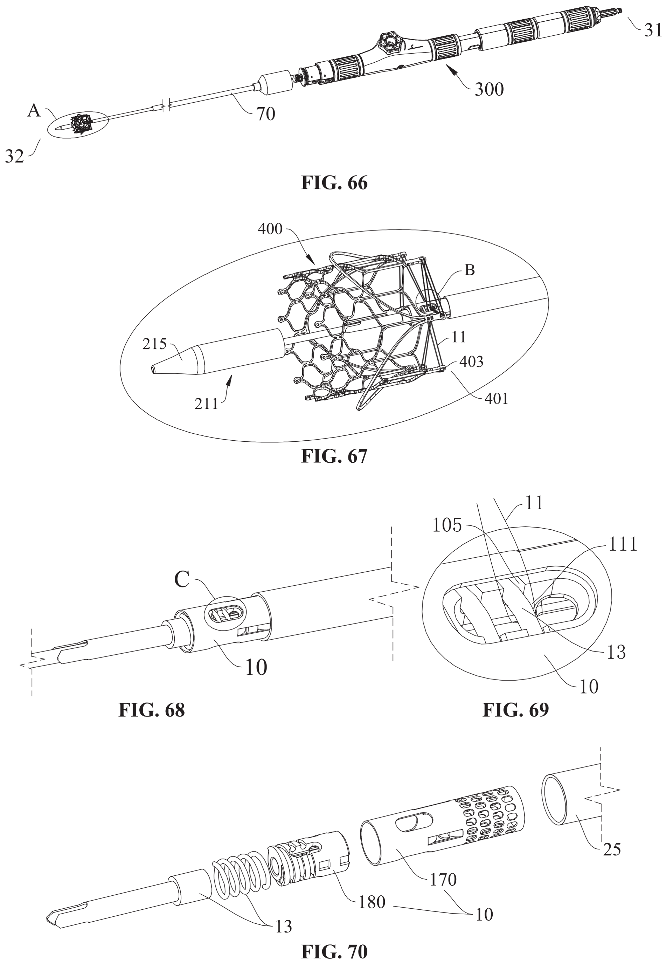

Referring to Figs. 66 to 80, the present application also provides a control mechanism for an artificial implant, including a base 10, a pulling wire 11, and a locking member 13. Referring to the aforementioned embodiments, the base 10 includes multiple components that are fixed to each other, and two of the multiple components are enclosed at their joint to form a locking hole 105. In this embodiment, the locking hole 105 is formed by the cooperation of two components, which can be independently processed to select suitable materials, eliminating the need for conventional drilling operations and reducing the overall processing difficulty of the base 10.

In one embodiment, the base 10 includes an outer cylinder 170 fixedly mounted around an inner cylinder 180, and the outer cylinder 170 and the inner cylinder 180 enclose to form the locking hole 105. It can be understood that the locking hole 105 is formed by the cooperation of the outer cylinder 170 and the inner cylinder 180. After the assembly of the outer cylinder 170 and the inner cylinder 180, a complete locking hole 105 is formed at the joint of the outer cylinder 170 and the inner cylinder 180.

The cross-sectional profile of the locking hole 105 is not strictly limited to being continuous and uninterrupted, but at least it can maintain the locking member 13 from leaving the base 10 after entering the locking hole 105.

For example, the cross-sectional profile of the locking hole 105 is continuous, which means that the locking hole is closed in its own circumferential direction. A part of the locking hole is formed by the inner cylinder 180 and at least a part of the locking hole is open, while the outer cylinder 170 closes this open part. Of course, the processing and fitting errors between the inner cylinder 180 and the outer cylinder 170 are ignored.

For example, the cross-sectional profile of the locking hole 105 is discontinuous, and a part of the locking hole is formed by the inner cylinder 180 and configured with an open portion, which is partially closed by the outer cylinder 170.

The cross-sectional shape of the locking hole 105 is not strictly limited, at least it is convenient for the locking member to engage or disengage, which may be, for example, circular, elliptical, arched, sealed U-shaped, etc., and may also be selected to correspond to the cross-sectional shape of the locking member. The outer cylinder 170 and the inner cylinder 180 may each provide a portion of the edge of the locking hole 105, for example, for example, each providing half or one providing the majority.

For example, the cross-sectional shape of the locking hole 105 is a sealed U-shape, the outer cylinder 170 provides the transverse part for sealing, and the inner cylinder 180 provides the U-shaped part. For example, the cross-sectional shape of locking hole 105 is arched, the outer cylinder 170 provides the chord part and the inner cylinder 180 provides the arc part. The outer cylinder 170 and the inner cylinder 180 can be nested as a whole or partially nested. The locking hole 105 can be located radially between the outer cylinder 170 and the inner cylinder 180, or it can be a stepped structure with axial (or angled) abutment between the outer cylinder 170 and the inner cylinder 180. The locking hole 105 is located on the stepped surface in the axial direction.

For example, as shown in Figs. 68-73, a part of the inner cylinder 180 is located outside the outer cylinder 170 and is turned radially and outwardly to form the stepped structure. The outward turning part abuts the end surface of the outer cylinder 170 in the axial direction, and the locking hole 105 is located at the abutment position.

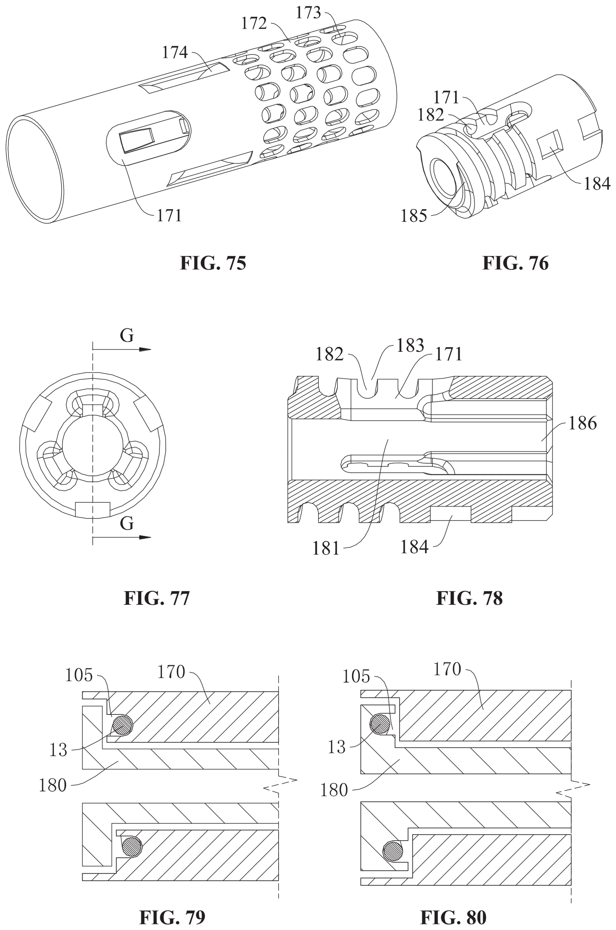

For example, as shown in Fig. 79 and Fig. 80, the stepped structure is located on the inner wall of the outer cylinder 170, and abuts a part of the end surface or outer circumference of the inner cylinder 180 (also configured with a stepped structure)in the axial direction. The locking hole 105 is located at the abutment position.

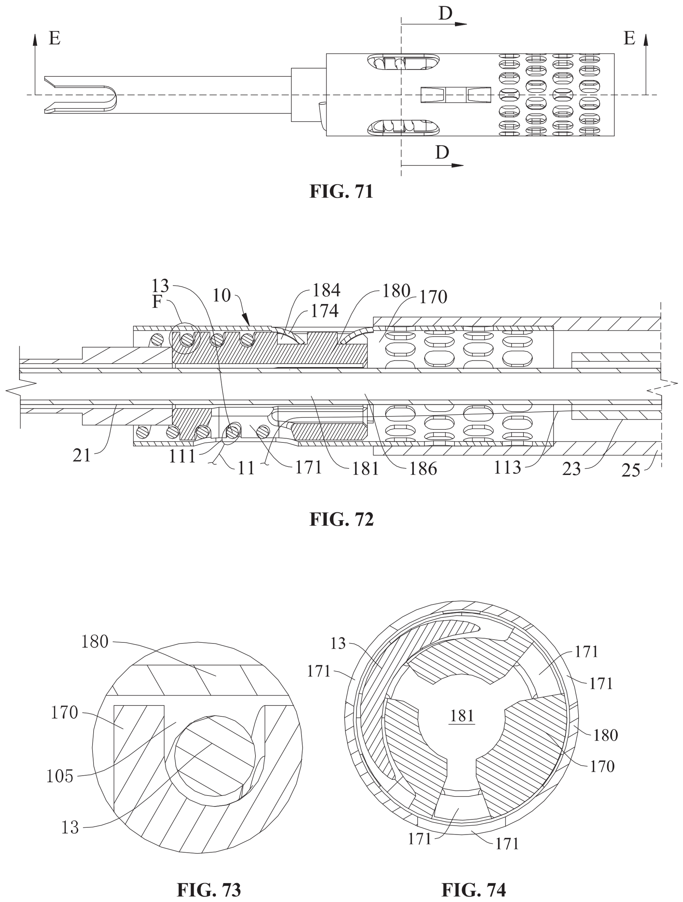

Referring specifically to Fig. 72, in one embodiment, the control mechanism further includes three shafts, namely a first shaft 21, a second shaft 23, and a third shaft 25, which are slidably connected in sequence from the inside to the outside. The base 10 is connected to the distal end of the third shaft 25, the pulling wire 11 is connected to the distal end of the second shaft 23, and the locking member 13 is connected to the distal end of the first shaft 21. The base 10 is penetrated along its axial direction and forms a first cavity 181 therein. The first shaft 21 extends through the first cavity 181 to the distal end of the base 10, for example, further extends beyond the distal end of the base 10.

The proximal end side of the base 10 has a first opening 186 communicated with the first cavity 181, and the outer circumferential surface (i.e., the circumferential side wall) of the base 10 has a second opening 171 communicated with the first cavity 181. One end of the pulling wire 11 (i.e. control end 113) extends out of the base 10 through the first opening 186 towards the proximal end, and the other end of the pulling wire 11 (i.e. free end 111) extends out of the base 10 through the second opening 171 to connect the artificial implant.

Referring to Figs. 66-85 again, there are multiple locking holes 105 arranged along the helical direction. The locking member 13 is inserted or removed from the locking holes 105 in sequence. In one embodiment, the locking holes 105 intersects with the second opening 171, which may be understood as that the second opening 171 extends along the radial direction of the base to form a first channel, and the locking holes 105 extend along the helical direction to form a second channel, wherein the first channel and the second channel intersect with each other to form an intersection portion (i.e., the helical intersects with the second opening 171). It can also be understood that all locking holes 105 are directly or indirectly communicated with each other through the second opening 171. The extension path of the pulling wire 11 passes through the intersection portion, and the locking member 13 enters and exits the locking hole 105 at the intersection portion to combine with the pulling wire 11.

Based on the specific structure of the base 10, the cross-sectional profile of the locking hole 105 at the rest of the base 10 except for the second opening 171 can be considered as a circumferential closure (i.e., a complete locking hole), while the cross-sectional profile of the locking hole 105 at the second opening (or intersection portion) is incomplete. At this time, the locking hole 105 has an opening, and the locking member 13 passes through the opening to engage into or disengage from the complete locking hole 105 during rotation.

As shown in Fig. 74, the second opening 171 of the inner cylinder 180 can be provided with a structure expanding radially and outwardly, which facilitates the insertion of the free end 111 of the pulling wire 11 during loading and also facilitates operation of the coordination and guidance tools.

In one embodiment, the inner cylinder 180 is an axial through structure, and its interior serves as the first cavity 181. According to the assembly relationship between the inner cylinder 180 and the outer cylinder 170, the outer wall of the inner cylinder 180 and the side wall of the outer cylinder 170 are provided with a second opening 171, and the inner wall is provided with a first opening 181. The opening size of the second opening 171 for the outer cylinder 170 and the inner cylinder 180 does not necessarily need to be the same, ensuring that the pulling wire 11 can pass through is sufficient. Among them, there are multiple pulling wires 11, and correspondingly there are multiple second openings 171 arranged at intervals along the circumferential direction of the base. The open side of locking hole 105 is oriented in the circumferential direction of the base. The free end 111 of the pulling wire 11, which is in the first state, extends out of the base 10 through the corresponding second opening 171, passes through the artificial implant 400, and returns to the same second opening 171, or returns to the adjacent second opening 171 in the circumferential direction. As shown in Figs. 72 to 78, the inner wall of the outer cylinder 170 and/or the outer wall of the inner cylinder 180 are configured with a helical groove 182 for providing the locking hole 105, and the helical groove 182 intersects with the second opening 171. The outer circumferential surface of the inner cylinder 180 is configured with the helical groove 182, which provides the aforementioned helical line. The helical groove 182 has a radially outward slot 183, and the area where the outer cylinder 170 and the inner cylinder 180 are matched is a straight cylinder structure. After the assembly of the outer cylinder 170 and the inner cylinder 180, the outer cylinder 170 partially closes the slot 183 to form a complete locking hole 105, which is used to limit the rotation path of the locking member 13 and avoid displacement of the locking member 13 during rotation. The cross-sectional shape of helical groove 182 refers to the cross-sectional shape of locking hole 105, such as being arc portion of the U-shaped or arched structure. For small-sized components such as the base 10, the above structural design facilitates the processing of the outer cylinder 170 and the inner cylinder 180, which can be made of metal or plastic materials. For example, the outer cylinder 170 is a tubular structure and is processed using a metal tube, while the inner cylinder 180 is injection molded from plastic. Correspondingly, the size of the second opening of the inner cylinder 180 is smaller than that of the second opening of the outer cylinder 170. The size of the second opening includes but is not limited to the length along the axial direction of the base and the width extending around the axis of the base. In addition, compared to existing drilling methods, the machining accuracy of the helical groove 182 has also been improved, correspondingly improving the smoothness of rotation of locking member 13.

In one embodiment, the proximal end side of the outer cylinder 170 is an extension section 172 that extends beyond the inner cylinder 180, and the extension section 172 is used to connect the third shaft 25 which extends towards the proximal end. There is a positioning structure provided between the outer cylinder 170 and the inner cylinder 180 to maintain their relative fixation. Based on the attached drawings, the positioning structure includes a positioning groove 184 defined in an outer wall of the inner cylinder 180, and a positioning block 174 formed on an inner wall of the outer cylinder 170 and embedded into the positioning groove 184. The positioning block 174 is a flange formed by stamping the side wall of the outer cylinder 170.

The outer cylinder 170 is a tubular structure, and the third shaft 25 is a pipe. The third shaft 25 and the extension section 172 are mutually overlapped and connected, such as fusion or bonding. In one embodiment, the side wall of the extension section 172 has a connecting hole 173, and adhesive or the like can be added into the connecting hole 173 to improve the connection strength between the third shaft 25 and the extension section 172 to avoid an increase in the radial size of the two after connection.

In one embodiment, the helical line is a cylindrical helical line, a conical helical line, or partially a conical helical line. Preferably, the helical line is a cylindrical helical line, and correspondingly, the outer cylinder 170 and the inner cylinder 180 are generally straight tube structures.

In one embodiment, the helical groove 182 extends to and open at the distal end surface of the inner cylinder 180, and the open portion of the helical groove 182 at the distal end surface of the inner cylinder 180 is provided with a guiding slope 185 to guide the locking member 13 to be assembled into the helical groove 182.

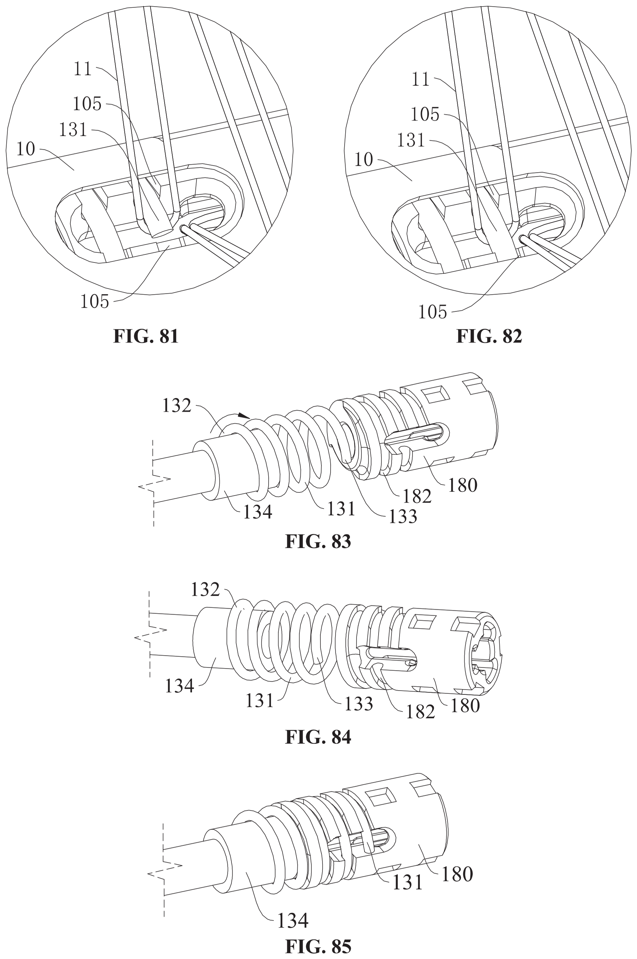

Referring to Figs. 81-85, in one embodiment, the locking member 13 has a positioning portion 131 that engages into or disengages from the locking hole 105 during rotation. The positioning portion 131 is a helical structure that matches the helical, improving the smoothness of rotation. The helical structure is wound at least once, for example, the helical structure is a multi-turn structure, as shown in the drawings, the helical structure winds 4-5 turns, which are arranged along the axial direction of the base. The extension path of the helical structure includes cylindrical helical, conical helical, or partial conical helical. The connecting portion 134 is movably mounted around the distal end of the first shaft 21, and can be axially separated from the distal end of the first shaft 21. Specifically, the connecting portion 134 is movable along the axial direction relative to the first shaft 21. In actual use, the first shaft 21 slides along the axial direction of the base to engage with or disengage from the connecting portion 134. After the first shaft 21 is axially engaged with the connecting portion 134, it drives the first shaft 21 to rotate to drive the connecting portion 134 to rotate, thereby driving the locking member 13 into or out of the locking hole 105. After the connecting portion 134 is disengaged from the distal end of the first shaft 21 in the axial direction, the control of the locking member 13 by the first shaft 21 is released. The specific coordination and control relationship between the first shaft 21 and the locking member 13 can be referred to the following embodiments.

In the rotation direction, the locking member 13 has an insertion direction and an exit direction. In one embodiment, a limit mechanism is provided between the locking member 13 and the base 10 to restrict the rotation of the locking member 13 in the insertion direction. Specifically, during the rotation of locking member 13, when the limit mechanism is activated, the locking member 13 is restricted from continuing to rotate in the insertion direction.

In the drawings, the limit mechanism includes a first end surface set at the proximal end of the connecting portion, and a second end surface set at the distal end of the base 10, wherein the second end surface abuts the first end surface. Specifically, the second end surface is set on the inner cylinder 180.

For example, the limit mechanism includes a head end surface set on the positioning portion 131, and a third end surface set on the base 10 that is in contact with the head end surface. Specifically, the third end surface is set on the side wall of the second opening 171 of the inner cylinder 180.

In one embodiment, the connecting portion 134 is a tubular shape with constant diameter or at least expands radially and outwardly at its proximal end, wherein the proximal end of the connecting portion 134 is fixedly connected to the positioning portion 131. Below is an explanation of the assembly process of the pulling wire 11, the base 10, and the locking member 13 outside the body:

First, the free end 111 of the pulling wire 11 enters the first cavity 181 through the first opening 186, and then sequentially extends through the second openings 171 of the inner cylinder 180 and the outer cylinder 170 to extend out of the base, passing through and winding around the artificial implant 400 and then back to the corresponding second opening 171, and at the same time, the locking member 13 is rotated to insert into the corresponding locking hole and combine with the corresponding pulling wire 11. The pulling wire 11 exposed out of the base 10 is retracted to pull the artificial implant 400 to deform radially approach to the base 10.

In one embodiment, the artificial implant 400 is a cylindrical structure with corresponding a circumferential direction. Multiple pulling wires 11 are configured, and the control ends 113 of the pulling wires 11 can be independently controlled or synchronously controlled to move. The positions where the pulling wires 11 interact with the artificial implant 400 are arranged at intervals along the circumferential direction of the artificial implant 400, which can improve the synchronization of the contraction and expansion of the artificial implant 400. For example, as shown in Fig. 67, there are three pulling wires 11 that interact with the artificial implant 400 at three locations which are arranged at intervals in the circumferential direction of the artificial implant.

The base 10 has a locking area, which is the area where the pulling wire 11 is combined with the locking member 13. For example, the locking area is the area where the second opening 171 of the aforementioned base 10 is located. There are multiple locking areas, wherein a number of the locking areas may be the same as or twice the number of pulling wires 11. The free ends 111 of the pulling wires 11 in the first state extends out of the base 10 through the current locking area, and returns to the same locking area or to an adjacent locking area by wrapping around the artificial implant 400. The free end 111 of each pulling wire 11 in the first state corresponds to one or two locking areas, avoiding interference between the pulling wires 11.

The control ends 113 of the pulling wires 11 may extend directly and independently to the control handle, or indirectly transmitted through intermediate components to reduce the risk of multiple pulling wires 11 intertwining with each other, as shown in Fig. 72. The second shaft 23 is a tubular structure and serves as the intermediate component, and the control ends 113 of the pulling wires 11 are connected to the second shaft 23.

Referring to Figs. 85 to 88, combined with the structure of the locking member 13 mentioned above, in one embodiment, at least a part of the locking member 13 is configured as the connecting portion 134. The first shaft 21 and the connecting portion 134 are selectively matched through a linkage assembly to drive the locking member 13 to rotate relative to the base 10. The two mating portions are configured as a linkage key and a linkage groove, wherein the linkage key 1341 protrudes radially from one of the connecting portion 134 and the first shaft 21, and the linkage groove 2111 is set on the other of the connecting portion 134 and the first shaft 21 and matched with the linkage key 1341. The linkage key 1341 and the linkage groove 2111 are formed at the end surface of the mating portion where they are located. In the drawings, the linkage key 1341 is set at the distal end of the connecting portion 134, and the linkage groove 2111 is set on the loading section 211. The rotational linkage between the linkage key 1341 and the linkage groove 2111 is manifested as the side wall of the linkage groove 2111 being in contact with the side wall of the linkage key 1341 in the circumferential direction. The connecting portion 134 is a cylindrical structure, and the linkage key 1341 is a sheet-like structure. In one embodiment, there are 2-4 linkage keys 1341 arranged along the circumferential direction, for example, there are two linkage keys 1341.

Preferably, the open side of the linkage groove 2111 is provided with an expansion structure for guiding the insertion of the linkage key 1341 into the linkage groove 2111, and the distal end surface of the linkage key 1341 is provided with a contraction structure for guiding the linkage key 1341 to insert into the linkage groove 2111, facilitating the combination of the linkage key 1341 and the linkage groove 2111.

In the engaged state, the loading section 211 is nested with the connecting portion 134. The inner circumference of the loading section 211 is provided with a support member 212, which is fixed to the distal end of the first shaft 21. One of the mating portions (i.e., the linkage groove) is provided on the support member 212. A limit structure is provided between the support member 212 and the loading section 211 to restrict their axial separation.

The loading section 211 has a guiding head 215 located at the distal end and connected to the first shaft 21, and a loading portion 216 connected to the guiding head 215 for accommodating the distal portion of the artificial implant. The support member 212 is set inside the guiding head 215.

Among them, the locking member will move towards the distal end during the rotation unlocking process. In one embodiment, when the loading section 211 and the locking member 13 are in the engaged state, there is an axial gap L1 between the linkage key and the linkage groove for the movement of the connecting portion towards the distal end. Similarly, the proximal end surface of the guiding head has the same axial gap L1 from the positioning portion 131. The axial gap L1 is greater than the movement distance of the locking member 13 towards the distal end.

Referring also to Fig. 89, an embodiment of the present application also provides a loading method for an artificial implant based on pulling wire control, including: providing the control mechanism of the aforementioned embodiment, in which the free end of the pulling wire passes through the proximal end of the artificial implant, and then combines with the locking member and is bound to the base by the locking member, the pulling wire has a control end opposite to the free end, by means of moving the control end, the exposed part of the pulling wire out of the base gradually contracts and drives the proximal end of the artificial implant to deform in the radial direction; providing a sheath tube surrounding the artificial implant and moving relative to the artificial implant from the proximal end to the distal end until the sheath tube completely envelops the artificial implant. The specific operation is carried out outside the body. First, the free end of the pulling wire is threaded through the proximal end of the artificial implant (such as the hole), and then the free end is guided through an instrument (such as a wire holding device 500 configured with two fork arms 522 arranged side by side at its distal end) and inserted into the locking area of the base. The first shaft is driven to make the loading section move towards the proximal end to complete the engagement with the locking member, at this time, the loading section is located inward of the artificial implant in the radial direction. The first shaft is then driven to rotate again, driving the locking member to rotate through the free end and enter the locking hole of the base, completing the restraint of the pulling wire. The first shaft and the loading section are pushed to the distal end until the loading section exits the artificial implant, and the control end of the pulling wire is driven to move towards the proximal end to compress and fold the proximal end of the artificial implant.

By using instruments such as grippers to compress the distal end of the artificial implant (the distal end of the inner frame), the loading section is driven to move towards the proximal end and wrap around the distal end of the inner frame, and then the artificial implant and sheath are moved relative to each other, such as driving other components outside the sheath tube to move towards the proximal end relative to the sheath tube, or pushing the sheath tube towards the distal end until the sheath tube completely wraps around the artificial implant.

Among them, the pre-loading operation is between the pulling wire, the artificial implant, and the control mechanism. An embodiment of the present application also provides a pre-loading method for an artificial implant based on pulling wire control, including: providing any of the above control mechanisms, in which the free end of the wire passes through the proximal end of the artificial implant; the free end 111 of the pulling wire 11 being configured with a wire loop and having two support points 118, which can be supported by two fork arms 522, a mounting section 119 being formed between the two support points 118 and placed into the locking area of the base 10, so that the wire loop is wrapped around the movement path of the locking member 13, which can be completed through a wire holding device 500; driving the locking member 13 to rotate along its own motion path to pass through the wire loop and insert into the locking hole of the base, so as to restrain the free end of the pulling wire. This operation refers to the combination steps of the pulling wire and the locking member in the aforementioned loading method for an artificial implant.

The various technical features of the above embodiments can be combined in any way. In order to make the description concise, not all possible combinations of the technical features in the above embodiments have been described. However, as long as there is no contradiction in the combination of these technical features, they should be considered within the scope of this specification. When the technical features of different embodiments are reflected in the same figure, it can be regarded that the figure also discloses the combination examples of the various embodiments involved.

The above embodiments only express several embodiments of the present application, and their descriptions are more specific and detailed, but should not be understood as limiting the scope of the patent application. It should be pointed out that for those ordinary skilled in the art, modifications and improvements can be made without departing from the concept of this application, which should be within the protection scope of this application.

## Claims

1. A control mechanism for an artificial implant, comprising:
a base having a locking hole;
a pulling wire having a free end capable of passing through and winding around the artificial implant or of being detached therefrom; and
a locking member rotatably engaged with the base, wherein during rotation, the locking member engages or disengages the locking hole to lock or unlock the free end of the pulling wire.

2. The control mechanism of claim 1, wherein the free end of the pulling wire has a first state being restricted to the base and a second state being released from the base, the free end comprises a ring, and a part of the locking member is configured as a positioning portion with a helical structure; and wherein
when the free end is in the first state, the positioning portion extends into the ring; and
when the free end is in the second state, the positioning portion exits the ring.

3. The control mechanism of claim 1, wherein the locking member in whole is located at a distal end of the base.

4. The control mechanism of claim 1, wherein the locking member and the base have a locking state in which they cooperate with each other and an unlocking state in which the cooperation is released, and in the unlocking state, the locking member is capable of further moving towards the distal end of the base relative to the locking state.

5. The control mechanism of claim 1, wherein the base is configured with a plurality of locking holes which are arranged offset from each other in an axial direction.

6. The control mechanism of claim 5, wherein the plurality of locking holes are arranged along a helical path.

7. The control mechanism of claim 6, wherein the locking member generates an axial displacement during its rotation and enters each locking hole sequentially.

8. The control mechanism of claim 7, wherein the locking member and the base have a locking state in which they cooperate with each other and an unlocking state in which the cooperation is released, and compared to the locking state, the locking member in the unlocking state further moves towards the distal end during its rotation.

9. The control mechanism of claim 1, wherein the base comprises a plurality of components fixed to each other, and two of the plurality of components are enclosed at their joint to form the locking hole.

10. The control mechanism of claim 9,
wherein the base has a radial direction, at least parts of the two of the plurality of components are in contact with each other in the radial direction, and the locking hole is located at a position where the two of the plurality of components are in contact in the radial direction.

11. The control mechanism of claim 9, wherein the base has an axial direction, at least parts of the two of the plurality of components are in contact with each other in the axial direction, and the locking hole is located at a position where the two of the plurality of components are in contact in the axial direction.

12. The control mechanism of claim 9, wherein an interior of the base defines a first cavity, a proximal end side of the base defines a first opening communicating with the first cavity, and an outer circumferential surface of the base defines a second opening communicating with the first cavity.

13. The control mechanism of claim 12, wherein the locking hole intersects with the second opening, and an intersection of the locking hole and the second opening serves as an open side of the locking hole for the locking member to engage into or disengage from the locking hole.

14. The control mechanism of claim 1, wherein the base comprises an inner cylinder and an outer cylinder mounted around and fixed to the inner cylinder, and the locking hole is provided radially between the outer cylinder and the inner cylinder.

15. The control mechanism of claim 14, wherein an interior of the base defines a first cavity, and the inner cylinder is an axial through structure to form the first cavity.

16. The control mechanism of claim 15, wherein a proximal end side of the base defines a first opening communicating with the first cavity, an outer circumferential surface of the base defines a second opening communicating with the first cavity, the base is configured with a plurality of locking holes arranged along a helical path which intersects with the second opening, and all of the locking holes are communicated with each other through the second opening directly or indirectly.

17. The control mechanism of claim 16, wherein an inner wall of the outer cylinder and/or an outer wall of the inner cylinder is/are configured with a helical groove for providing the locking holes, and the helical groove intersects with the second opening.

18. The control mechanism of claim 14, wherein the outer cylinder and the inner cylinder each are made of metal or plastic.

19. The control mechanism of claim 14, wherein a proximal end side of the outer cylinder is configured as an extension section extending beyond the inner cylinder, and the extension section is configured to connect a third shaft which extends towards a proximal end.

20. The control mechanism of claim 19, wherein a side wall of the extension section is configured with a connecting hole for connecting the third shaft.

21. The control mechanism of claim 14, wherein positioning structures that cooperates with each other are provided between the outer cylinder and the inner cylinder.

22. The control mechanism of claim 21, wherein an outer wall of the inner cylinder is configured with a positioning groove, and an inner wall of the outer cylinder is configured with a positioning block which is engaged into the positioning groove.

23. The control mechanism of claim 22, wherein a part of a side wall of the outer cylinder deforms to form the positioning block.

24. The control mechanism of claim 17, wherein the helical groove extends to a distal end surface of the inner cylinder.

25. The control mechanism of claim 24, wherein a portion of the helical groove at the distal end surface of the inner cylinder is configured with a guiding slope to guide the locking member to be assembled into the helical groove.

26. A control mechanism for an artificial implant, comprising:
a base configured with a locking area;
a pulling wire having a free end being capable of passing through or of being detached therefrom; and
a locking member being rotatably engaged with the base, wherein the locking member comprises a positioning portion that engages into or disengages from the locking area during its rotation, and the positioning portion cooperates with the free end of the pulling wire to restrict the artificial implant.

27. The control mechanism of claim 26, wherein the base has a distal end and a proximal end opposite to each other, and an axial direction extending between the distal end and proximal end, an interior of the base defines a first cavity, at least a part of the locking member is configured as a connecting portion located in the first cavity, and the positioning portion further extends from the connecting portion to the distal end.

28. The control mechanism of claim 26, wherein the base has a distal end and a proximal end opposite to each other, and an axial direction extending between the distal end and proximal end, at least a part of the locking member is configured as a connecting portion located at a distal end side of the base, and the positioning portion further extends from the connecting portion to the proximal end.

29. The control mechanism of claim 26, wherein
an interior of the base defines a first cavity, a proximal end side of the base defines a first opening communicating with the first cavity, and an outer circumferential surface of the base defines a second opening communicating with the first cavity; and
the pulling wire extends through the first cavity, one end of the pulling wire extends out of the base through the first opening towards the proximal end, and another end of the pulling wire (the free end) extends out of the base through the second opening for connecting the artificial implant.

30. The control mechanism of claim 29, wherein the locking area is located in the second opening, helical plurality of second openings arranged at intervals along a circumferential direction of the base, and ribs are provided between adjacent second openings.

31. The control mechanism of claim 30, wherein all of the ribs are converged and fixed to each other at a distal end of the base.

32. The control mechanism of claim 31, wherein all of the ribs are converged to form an annular structure.

33. The control mechanism of claim 30, wherein at least one of the ribs is provided with a locking hole, and there is/are one locking hole or a plurality of locking holes on the same rib.

34. The control mechanism of claim 33, wherein an orientation of an open direction of the locking hole is the circumferential direction of the base.

35. The control mechanism of claim 33, wherein all of the ribs are configured with the locking hole.

36. The control mechanism of claim 33, wherein helical plurality of ribs configured with the locking hole, and the positioning portion as a whole has a tail end and a head end that are opposite to each other, and the head end is engaged into or disengaged from each locking hole sequentially along with the rotation of the locking member.

37. The control mechanism of claim 33, wherein the locking holes are arranged in sequence along an extension direction of the rib where they are located.

38. The control mechanism of claim 29, wherein the free end of the pulling wire has a first state being restricted to the base and a second state being released from the base, and when the cooperation between the locking member and the free end of the pulling member is released, a restriction on the artificial implant is released;
in the first state, the free end extends out of the base through a corresponding second opening, winds around the artificial implant, and returns to the locking area corresponding to the same second opening or returns to the locking area corresponding to an adjacent second opening.

39. The control mechanism of claim 38, wherein the free end of the pulling wire in the first state is located in a current locking area, and two ends of the positioning portion located in the current locking area are inserted into the locking holes at corresponding sides, respectively.

40. The control mechanism of claim 33, wherein the positioning portion has a curved shape and extends circumferentially around the base, and the positioning portion is engaged into or disengaged from the locking hole along with the rotation of the locking member.

41. The control mechanism of claim 26, wherein the positioning portion comprises at least one arc-shaped structure that matches with the base.

42. The control mechanism of claim 26, wherein the positioning portion is configured as a helical structure.

43. The control mechanism of claim 42, wherein the helical structure is wound at least one turn.

44. The control mechanism of claim 43, wherein the helical structure is configured with a plurality of turns which are arranged along an axial direction.

45. The control mechanism of claim 42, wherein the helical structure is a cylindrical helical or at least partially a conical spiral.

46. The control mechanism of claim 42, wherein the positioning portion as a whole has a tail end and a head end being opposite to each other along a winding direction of the helical structure, and a connecting portion is fixed to one of the tail end and head end of the positioning portion.

47. The control mechanism of claim 27 or 28, wherein the connecting portion is tubular-shaped, and a distal end of the positioning portion is mounted around an outer circumference of the connecting portion or abuts against an end portion of the connecting portion.

48. The control mechanism of claim 47, wherein the free end of the pulling wire has a first state being restricted to the base and a second state being released from the base; when the cooperation between the locking member and the free end of the pulling member is released, a restriction on the artificial implant is released; in the first state, a limit mechanism is provided between the locking member and the base to prevent the locking member from rotating in an insertion direction.

49. The control mechanism of claim 48, wherein the limit mechanism comprises a first end surface provided at a proximal end of the connecting portion, and a second end surface provided at a distal end of the base, and the second end surface abuts the first end surface.

50. The control mechanism of claim 28, wherein the connecting portion is tubular-shaped with constant diameter or at least expands radially and outwardly at its proximal end, the proximal end of the connecting portion is configured as a connecting section, a distal end of the base comprises an extending section, and the connecting section and the extending section are nested with each other.

51. The control mechanism of claim 26, wherein the free end of the pulling wire has a first state being restricted to the base and a second state being released from the base; the pulling wire further comprises a control end opposite to the free end, the control end is movable relative to the base;
when the free end is in the first state, a length of the pulling wire exposed out of the base is adjusted by operating the control end; and
when the free end is in the second state, the pulling wire is driven to detach from the artificial implant by operating the control end.

52. The control mechanism of claim 51, wherein a plurality of pulling wires is configured, the free ends of the pulling wires move independently of each other, while the control ends of the pulling wires move synchronously.

53. The control mechanism of claim 52, wherein the control ends of the pulling wires are connected to a second shaft.

54. The control mechanism of claim 51, wherein the free end has a ring,
when the free end is in the first state, the positioning portion extends into the ring, and
when the free end is in the second state, the positioning portion exits the ring.

55. The control mechanism of claim 54, wherein the ring is independently configured or wound by the pulling wire.

56. The control mechanism of claim 26, wherein the free end of the pulling wire has a first state being restricted to the base and a second state being released from the base;
in the first state, the pulling wire extends out of the base through a current locking area, winds around a free end of the artificial implant, and returns to the same locking area or an adjacent locking area.

57. The control mechanism of claim 1 or 26, wherein the artificial implant has an axial direction, one end of the artificial implant in the axial direction is configured as a wire control end, the wire control end defines a hole for extending of the pulling wire, and according to a degree of deformation, the artificial implant comprise:
a folding state, wherein the wire control end is radially folded and close to the base;
an expanding state, wherein the wire control end expands radially and away from the base;
wherein a round-trip path of the same pulling wire around the artificial implant does not repeated.

58. The control mechanism of claim 57, wherein there is a plurality of holes arranged at intervals, and in the expanding state, the same pulling wire extends through at least two holes.

59. The control mechanism of claim 57, wherein the round-trip path of the same pulling wire around the artificial implant is substantially triangular-shaped.

60. The control mechanism of claim 57, wherein a number of the holes is twice the number of the pulling wires, and in the expanding state, each pulling wire corresponds to two holes.

61. The control mechanism of claim 1 or 26, wherein the control mechanism further comprises a first shaft configured for driving the locking member to rotate relative to the base.

62. The control mechanism of claim 61, wherein a distal end of the first shaft movably penetrates the base, and the locking member is movably mounted around the first shaft and selectively linked with the first shaft.

63. The control mechanism of claim 62, wherein the pulling wire further comprises a control end opposite to the free end, the control end is movable relative to the base, and the control mechanism further comprises a second shaft connected to the control end.

64. The control mechanism of claim 63, wherein the second shaft is tubular-shaped and movably mounted around the first shaft.

65. The control mechanism of claim 64, wherein the control mechanism further comprises a third shaft, the base is connected to a distal end of the third shaft, and the third shaft is movably mounted around the first shaft.

66. The control mechanism of claim 65, wherein the second shaft is slidably mounted around the first shaft, and the third shaft is slidably mounted around the second shaft.

67. The control mechanism of claim 61, wherein a loading section is fixed to a distal end of the first shaft, and the loading section is open towards the proximal end for accommodating a distal portion of the artificial implant; the loading section is configured as a radially deformable structure to adapt to being wrapped by the artificial implant when the artificial implant is withdrawn.

68. A control mechanism having a distal end and a proximal end being opposite to each other and an axial direction extending between the distal end and the proximal end, comprising:
intervention components, comprising a first intervention component and a second intervention component;
a transmission member, a distal end if the transmission member being fixedly connected to the first intervention component;
a control handle, being connected to and controlling a proximal end of the transmission member; and
a linkage structure, comprising two mating portions being selectively linked, one of which is fixed to the second intervention component and the other is fixed to the transmission member, and a linkage state of the two mating portions being switched during the movement of the transmission member.

69. The control mechanism of claim 68, wherein there is a relative position wherein the first intervention component and the second intervention component are partly nested with each other.

70. The control mechanism of claim 68, wherein the first intervention component and the second intervention component each comprise:
an inner shaft assembly, configured for carrying an artificial heart valve before release;
a sheath tube, being capable of wrapping around the artificial heart valve before release;
a base being connected to the artificial heart valve before release and axially limited to each other;
a locking member configured for limit the artificial heart valve before release in the base; and
a pulling wire connected to the artificial heart valve, configured for controlling the release process of the artificial heart valve.

71. The control mechanism of claim 68, wherein the control mechanism further comprises a balloon body for carrying the artificial heart valve and expanding the artificial heart valve.

72. The control mechanism of claim 68, wherein the two mating portions are linked or unlinked when the transmission member rotates.

73. The control mechanism of claim 68, wherein the two mating portions are linked or unlinked when the transmission member slides along the axial direction.

74. The control mechanism of claim 68, wherein the two mating portions drive the second intervention component to rotate or move linearly when they are linked.

75. A locking mechanism for connecting artificial implant with delivery system, comprising:
a first shaft, wherein a loading section is fixed to a distal end of the first shaft and opens towards a proximal end, configured for accommodating a distal portion of the artificial implant;
a pulling wire configured with a free end being capable of passing through and winding around the artificial implant or of being detached therefrom;
a third shaft being slidably mounted around the first shaft;
a locking assembly, wherein the artificial implant is bound to the locking assembly by the pulling wire during loading, the locking assembly comprising a locking member and a base fixed to the third shaft, the locking member and the base having:
a locking state in which they cooperate with each other to restrict the detachment of the pulling wire from the artificial implant; and
an unlocking state in which the cooperation is released to allow the pulling wire to detach from the artificial implant; and
a linkage assembly, acting between the first shaft and the locking member to selectively link the first shaft and the locking member; wherein
in the unlocking state, the locking member is rotatably mounted around the first shaft; and
in the locking state, the locking member is linked with the first shaft and rotates along with the first shaft until it is combined with the base and binds the pulling wire.

76. The locking mechanism of claim 75, wherein the linkage assembly comprises two mating portions, one of which is connected to the loading section and the other is connected to the locking member, and axial movement of the loading section cause the two mating portions to be linked or unlinked.

77. The locking mechanism of claim 76, wherein the two mating portions are configured as a linkage key and a linkage groove which are capable of being separated by axially sliding, and the linkage key is rotatably linked with the linkage groove when it is inserted into the linkage groove.

78. The locking mechanism of claim 77, wherein there is a plurality of linkage connected to each other through a tubular component, and the plurality of linkage keys are arranged along a circumferential direction of the tubular component.

79. The locking mechanism of claim 77, wherein there is a plurality of linkage grooves connected to each other through a tubular component, and the plurality of linkage grooves are arranged along a circumferential direction of the tubular component.

80. The locking mechanism of claim 77, wherein at least a part of the locking member is configured as a connecting portion, and the linkage key or the linkage groove is set at a proximal end or a distal end of the connecting portion.

81. The locking mechanism of claim 80, wherein the connecting portion is located inside or outside the base.

82. The locking mechanism of claim 80, wherein the connecting portion is configured as a tubular structure, the linkage groove is opened on a pipe wall of the connecting portion, and the linkage key is provided on an inner circumferential wall of the loading section.

83. The locking mechanism of claim 77, wherein an open side of the linkage groove is configured with an expansion structure.

84. A loading method for an artificial implant based on wire control, comprising:
providing a control mechanism, in which a free end of a pulling wire passes through a proximal end of the artificial implant and then is combined with a locking member and is bound to the base by the locking member; wherein
the pulling wire has a control end opposite to the free end, and moving the control end makes a portion of the pulling wire exposed out of the base be shorten gradually to drive a proximal end of the artificial implant to deform radially and fold;
providing a sheath tube and making the sheath tune be around the artificial implant and move relative to the artificial implant from a proximal end to a distal end until the artificial implant are completely folded by the sheath tube.

85. A pre-loading method for an artificial implant based on wire control, comprising:
providing a control mechanism where a free end of a pulling wire passes through a proximal end of the artificial implant; wherein
the free end of the pulling wire is configured with a wire loop and two support points, a mounting section is formed between the two support points and placed in a locking area of the base, so that the pulling wire is around a movement path of the locking member;
driving the locking member to rotate along the movement path, pass through the wire loop and engage into a locking hole of the base, thereby securing bound the free end of the pulling wire.
